# EUROPEAN PATENT APPLICATION

(11) **EP 4 226 841 A1**
(43) Date of publication of application: **16.08.2023**
(21) Application number: 21877809.0
(22) Date of filing: 30.06.2021
(51) Int. Cl.: A61B 3/08, A61B 3/028, A61B 3/00, A61H 5/00, A61B 5/00, G16H 50/20

(54) **DEVICE AND METHOD FOR VIRTUAL-REALITY-BASED EYE HEALTH MEASUREMENT, AND SYSTEM THEREFOR**

(30) Priority: 07.10.2020 KR 20200129225; 07.10.2020 KR 20200129232
(71) Applicant: M2S Co.,Ltd, Seongnam-Si, Gyeonggi-do 13449 (KR)
(72) Inventor: LEE, Tae Hwi, Seoul 05502 (KR)
(74) Representative: Keilitz Haines & Partner Patentanwälte PartGmbB
(86) International application number: PCT/KR2021/008271
(87) International publication number: WO 2022/075554

(57) **Abstract**

A method for virtual-reality-based eye health measurement, according to an embodiment, is performed by a processor of an eye health measuring instrument, which is linked with a head-mounted display, the method comprising the steps of: providing an eye health condition survey interface; determining an eye health measurement method on the basis of the provided survey interface; measuring eye health conditions with the determined eye health measurement method; displaying the measurement result of the eye health conditions; and providing prescription content on the basis of the measurement result of the eye health conditions.

## Description

### BACKGROUND

### Field

The present invention relates to a device and method for virtual-reality-based eye health measurement, and a system therefor. More specifically, the present invention relates to a virtual reality-based eye health measurement device, method, and system for performing an eye health measurement process using virtual reality and providing prescription content and eye health solution services based on the result.

### Related Art

Modern people show abnormal signals related to eye health conditions depending on personal, environmental, and habitual factors.

For example, there are cases in which abnormal eyesight symptoms such as nearsightedness, farsightedness, and/or astigmatism according to deterioration of eye health conditions may be shown. The abnormal signal related to the eye health conditions may include not only an eyesight abnormal signal, but also a pupil abnormality, an eyeball motion abnormality, and the like.

In order to understand such eye health conditions, measurement of various parameters (for example, stereopsis, strabismus, binocular vision, double vision, pupil and/or field of view) is required. Recently, driven by the development of various technologies, introduction of a technology for measuring eye health conditions using a head-mounted display (HMD) device is actively progressing.

In detail, recently, it is a trend that gradually spreads to understand the eye health conditions of a measurement subject based on a virtual reality (VR) image based on the HMD.

However, in the conventional technical field, since the aforementioned HMD is used in close contact with the faces of a plurality of measurement subjects, infection with various bacteria or contaminants (for example, cosmetics) frequently occurs.

In addition, conventional methods of measuring an eye disease commonly involve manual labor in which a measurement performer (a doctor or an optician) uses expensive measurement equipment to measure the eye condition of a measurement subject.

Accordingly, depending on a measurement performer, the measurement result for a measurement subject is not objective, and it takes a long time. Furthermore, excessive measurement cost is incurred due to the labor cost of the measurement performer.

In addition, in order to measure stereopsis among eye diseases, professional equipment such as polarized glasses or polarizing filters is required, and special optotypes optimized for measuring stereopsis are further required. As such, the time and cost required to prepare a stereopsis measurement environment are considerable.

In addition, although it is true that the measuring equipment for measuring eyesight among eye diseases has been improved from a method using the conventional eyesight test chart, a measurement performer analyzes the measurement information of a measurement subject acquired through the measuring equipment again to determine the type of an eye disease. Accordingly, it is difficult to quickly determine an eye disease based on measurement information of the measurement subject.

In addition, in the case of measuring an eye disease of a measurement subject with measuring equipment, there is an inconvenience that the measurement subject should not move from a fixed position provided in the measuring equipment until the measurement is completed. In other words, since the measurement position of the eye disease of the measurement subject is dependent on the measuring equipment, the measurement environment of the measurement subject is not good, such as having to measure the eye disease in a rigid state.

In line with this trend, in recent various industrial fields, based on the HMD, which is a display device worn on the head, various augmented reality (AR) or virtual reality (VR) data related to the corresponding industrial field are generated and managed, and if necessary, communication is performed to exchange the generated data based on a wired or wireless network.

### SUMMARY

The present invention has been devised to obviate the above limitation. An aspect of the present invention is directed to providing a virtual reality-based eye health measurement device, method, and system for providing function operations that help keep a head-mounted display used for eye health measurement clean and assist convenient management.

In addition, an aspect of the present invention is directed to providing a virtual reality-based eye health measurement device, method, and system for determining an eye health measurement method customized for a measurement subject (in other words, a subject to be measured) based on a survey process related to eye health, and performing an eye health measurement service for the measurement subject based on the determined eye health measurement method.

In addition, an aspect of the present invention is directed to implementing a virtual reality-based eye health measurement device, method, and system for providing a graphic image of the acquired eye health condition measurement result for the measurement subject.

In addition, an aspect of the present invention is directed to implementing a virtual reality-based eye health measurement device, method, and system for providing an eye health condition measurement result of a measurement subject acquired based on virtual reality based on an eye health measurement computing device of the measurement subject (for example, a mobile type computing device and/or a desktop type computing device).

In addition, an aspect of the present invention is directed to implementing a virtual reality-based eye health measurement device, method, and system for providing an eye health solution service that assists customized eye health management optimized for a measurement subject from a diversified perspective based on the eye health condition measurement result provided as above.

A method for virtual reality-based eye health measurement according to an embodiment is performed by a processor of an eye health measuring instrument, which is linked with a head-mounted display, wherein the method includes: providing an eye health condition survey interface; determining an eye health measurement method based on the provided survey interface; measuring eye health conditions with the determined eye health measurement method; displaying the measurement result of the eye health conditions; and providing prescription content based on the measurement result of the eye health conditions.

In another aspect, a method for virtual reality-based eye health measurement according to an embodiment is performed by a processor of an eye health measuring instrument, which is linked with a head-mounted display, wherein the method includes: measuring eye health conditions of a measurement subject with a virtual reality-based eye health measurement method; controlling to display eye health condition information of the measurement subject, which is a result of the measured eye health conditions; determining an eye healing image, which is virtual reality content to be provided to the measurement subject, based on the generated eye health condition information; and controlling to output the determined eye healing image to the measurement subject.

In addition, a device for virtual reality-based eye health measurement according to an embodiment includes: an eye health measuring instrument providing a virtual reality-based eye health condition measurement service; and a head-mounted display outputting the virtual reality image, wherein the eye health measuring instrument includes: a body forming a body of the eye health measuring instrument; a sensor portion sensing an inflow or outflow of the head-mounted display to a head-mounted display accommodation portion disposed on the body; a disinfection portion disinfecting the head-mounted display introduced into the accommodation portion; and a processor controlling the sensor portion to acquire protection portion sensing information including at least one of position, area, direction, and angle information of a facial contact protection portion of the head-mounted display introduced into the accommodation portion, and tilting the disinfection portion toward the facial contact protection portion based on the acquired protection portion sensing information.

The virtual reality-based eye health measurement device, method, and system according to an embodiment of the present invention provide a functional operation (in an embodiment, a sterilization/disinfection function) to keep the head-mounted display used for eye health measurement clean. Accordingly, it is possible to safely maintain the cleanliness of the head-mounted display used for an eye health measurement service without checking a contamination state of the head-mounted display every time or manually performing a separate sterilization treatment.

In addition, the virtual reality-based eye health measurement device, method, and system according to an embodiment of the present invention provide a functional operation (in an embodiment, an automatic charging function) that assists in convenient management of the head-mounted display used for eye health measurement. Accordingly, it is possible to easily and conveniently manage eye health condition measurement environment.

In addition, the virtual reality-based eye health measurement device, method, and system according to an embodiment of the present invention determine an eye health measurement method customized for a measurement subject (in other words, a subject to be measured) based on a survey process related to eye health, and perform an eye health measurement service for the measurement subject based on the determined eye health measurement method. Accordingly, it is possible to acquire consistent and reliable base data capable of analyzing the eye health condition of the subject through a systematic measurement method, and to derive highly reliable analysis results based thereon.

In addition, the virtual reality-based eye health measurement device, method, and system according to an embodiment of the present invention understand the current eye health condition of a measurement subject based on the results of the eye health condition survey analysis, and select and provide an eye health measurement method necessary for the corresponding measurement subject based thereon. Accordingly, it is possible to conduct a customized eye health measurement process optimized for each subject, and thus, it is possible to reduce the cost or effort required to measure eye health conditions, as a result, the efficiency of the eye health measurement service can be improved.

In addition, the virtual reality-based eye health measurement device, method, and system according to an embodiment of the present invention implements and provides the eye health condition measurement result as a graphic image, so that the eye health condition measurement result can be recognized more intuitively.

In addition, the virtual reality-based eye health measurement device, method, and system according to an embodiment of the present invention provides the eye health condition measurement result of a measurement subject acquired based on virtual reality based on an eye health measurement computing device of the measurement subject (for example, a mobile type computing device and/or a desktop type computing device). Accordingly, it is possible to easily track, observe, and care for eye health conditions in life, and thus, it is possible to reduce the cost or effort required for eye health management.

In addition, the virtual reality-based eye health measurement device, method, and system according to an embodiment of the present invention provides an eye health solution service that assists customized eye health management optimized for a measurement subject from a diversified perspective based on the eye health condition measurement result. Accordingly, it is possible to conveniently manage and care for eye health in various aspects, and thus, it is possible to induce the measurement subject to participate more actively in eye health management also in daily life.

### BRIEF DESCRIPTION OF THE DRAWINGS

FIG. 1 is a conceptual diagram of a system for virtual reality-based eye health measurement according to an embodiment of the present invention.
FIG. 2 is an example of a state illustrating an eye health measurement device according to an embodiment of the present invention.
FIG. 3 is an internal block diagram of an eye health measuring instrument according to an embodiment of the present invention.
FIGS. 4 and 5 are examples of diagrams for explaining a disinfection portion according to an embodiment of the present invention.
FIG. 6 is an example of a state illustrating an eye health measurement device including a driving portion according to an embodiment of the present invention.
FIG. 7 is an example of a diagram for explaining a charging portion according to an embodiment of the present invention.
FIG. 8 is an exploded perspective view of a head-mounted display according to an embodiment of the present invention.
FIG. 9 is an internal block diagram of a head-mounted display according to an embodiment of the present invention.
FIG. 10 is an example of a diagram for explaining the operating principle of the eye health measurement device according to an embodiment of the present invention.
FIG. 11 is an internal block diagram of an eye health platform management server according to an embodiment of the present invention.
FIG. 12 is a flowchart illustrating a method for virtual reality-based eye health measurement according to an embodiment of the present invention.
FIGS. 13A and 13B are examples of an eye health condition survey interface according to an embodiment of the present invention.
FIGS. 14A and 14B are examples of states displaying eye health condition survey information according to an embodiment of the present invention.
FIGS. 15 and 16 are examples of diagrams for explaining stereopsis measurement content according to an embodiment of the present invention.
FIG. 17 is an example of a diagram for explaining a method of implementing a displacement optotype according to an embodiment of the present invention.
FIG. 18 is an example of a diagram for explaining eyesight measurement content according to an embodiment of the present invention.
FIGS. 19A and 19B are examples of states displaying eye health condition measurement results according to an embodiment of the present invention.
FIGS. 20 to 23 are examples of diagrams for explaining an eye healing image according to an embodiment of the present invention.
FIG. 24 is an internal block diagram of a mobile type computing device according to an embodiment of the present invention.
FIG. 25 is a flowchart illustrating a method for virtual reality-based eye health measurement according to an embodiment of the present invention.
FIG. 26 is an example of a state in which an eye health solution service is provided in a mobile type computing device according to an embodiment of the present invention.
FIG. 27 is a conceptual diagram for explaining a method of providing an eye health solution service in a mobile type computing device according to an embodiment of the present invention.
FIGS. 28A, 28B and 28C are examples of diagrams for explaining measurement report content according to an embodiment of the present invention.
FIG. 29 is an example of a diagram for explaining eye movement index content according to an embodiment of the present invention.
FIGS. 30A and 30B are examples of diagrams for explaining simple eye measurement content according to an embodiment of the present invention.
FIGS. 31A and 31B are examples of diagrams for explaining simple eye movement content according to an embodiment of the present invention.
FIG. 32 is an example of a diagram for explaining knowledge content related to eye health according to an embodiment of the present invention.
FIGS. 33A and 33B are examples of diagrams for explaining ophthalmic institution information content according to an embodiment of the present invention.
FIG. 34 is an example of a diagram for explaining expert consultation content according to an embodiment of the present invention.

### DESCRIPTION OF EXEMPLARY EMBODIMENTS

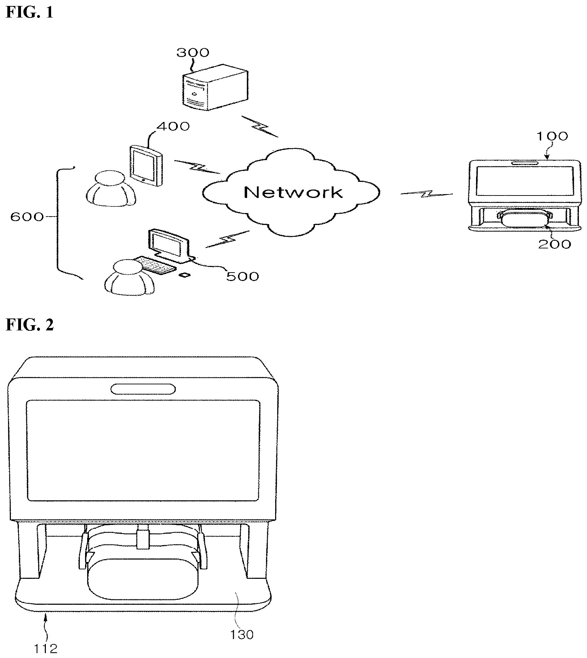

FIG. 1 is a conceptual diagram of a system for virtual reality-based eye health measurement according to an embodiment of the present invention.

Referring to FIG. 1, the system for virtual reality-based eye health measurement according to an embodiment of the present invention (hereinafter referred to as an eye health measurement system) may include an eye health measurement device (an eye health measuring instrument 100 and a head-mounted display 200), an eye health platform management server 300, and an eye health measurement computing device 600.

In an embodiment, the eye health measuring instrument 100, the head-mounted display 200, the eye health platform management server 300, and the eye health measurement computing device 600 may be linked to perform an eye health measurement process based on virtual reality, and to implement a virtual reality-based eye health measurement service (hereinafter referred to as an eye health measurement service) that provides prescription content and eye health solution services based on the result.

The eye health measuring instrument 100, the head-mounted display 200, the eye health platform management server 300, and the eye health measurement computing device 600 of FIG. 1 may be connected based on a network.

The network means a connection structure enabling information exchange among nodes such as the eye health measuring instrument 100, the head-mounted display 200, the eye health platform management server 300, and the eye health measurement computing device 600. For example, the network may include a 3GPP (3rd Generation Partnership Project) network, an LTE (Long Term Evolution) network, a WIMAX (World Interoperability for Microwave Access) network, the Internet, a LAN (Local Area Network), a Wireless LAN (Wireless Local Area Network), a WAN (Wide Area Network), a PAN (Personal Area Network), a Bluetooth network, a satellite broadcasting network, an analogue broadcasting network, a DMB (Digital Multimedia Broadcasting) network, etc., but is not limited thereto.

Hereinafter, the configuration and operation method of each of the eye health measuring instrument 100, the head-mounted display 200, the eye health platform management server 300, and the eye health measurement computing device 600 implementing the eye health measurement system will be explained in detail with reference to the accompanying drawings.

### - 1) Eye Health Measurement Device

FIG. 2 is an example of a state illustrating an eye health measurement device according to an embodiment of the present invention.

Referring to FIG. 2, the eye health measurement device according to an embodiment of the present invention may include the eye health measuring instrument 100 and the head-mounted display 200.

In an embodiment, the eye health measurement device including the eye health measuring instrument 100 and the head-mounted display 200 may provide an eye health measurement service for understanding eye health conditions of a measurement subject (hereinafter referred to as a subject) based on a virtual reality image output from the head-mounted display 200.

Hereinafter, the eye health measuring instrument 100 and the head-mounted display 200 included in the eye health measurement device as described above will be described in detail with reference to the accompanying drawings.

### <100: Eye Health Measuring Instrument >

The eye health measuring instrument 100 according to an embodiment of the present invention is a device capable of measuring eye health conditions of a subject based on a virtual reality image in conjunction with head-mounted display 200.

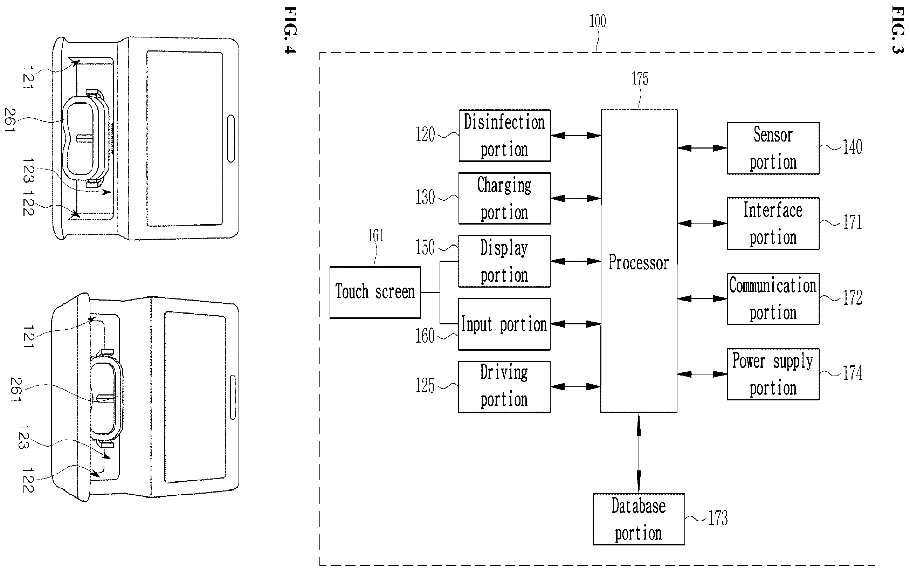

FIG. 3 is an internal block diagram of an eye health measuring instrument according to an embodiment of the present invention.

As illustrated in FIGS. 2 and 3, the eye health measuring instrument 100 according to an embodiment of the present invention may include a body 110, a disinfection portion 120, a charging portion 130, a sensor portion 140, and a display portion 150, and an input portion 160.

However, the components illustrated in FIGS. 2 and 3 are not essential for the eye health measuring instrument 100, so the eye health measuring instrument 100 may be implemented with more components or fewer components. Hereafter, the components are sequentially described.

In an embodiment, the body 110 of the eye health measuring instrument 100 forms an appearance as a body of the eye health measuring instrument 100, and is provided with various units necessary for driving the eye health measuring instrument 100 inside and outside. Can

In the body 110, a main body portion 111 on which the display portion 150 for outputting a predetermined graphic image is disposed may be formed on an upper side.

In an embodiment, the main body portion 111 may be implemented in the shape of a square box having a size capable of accommodating the display portion 150.

In addition, in the body 110 according to an embodiment, a base portion 112 for supporting the aforementioned main body portion 111 from the ground may be disposed on a lower side.

In this connection, the main body portion 111 may be spaced apart from the base portion 112 and the main body portion 111 so as to form a predetermined cavity region between the base portion 112 and the main body portion 111, and simultaneously, may be supported from the ground through a support portion 113 that connects and supports the main body portion 111 from the base portion 112.

Herein, the support portion 113 according to an embodiment may include a first support portion 113-1 for connecting and supporting one side (for example, a left end) of the base portion 112 and the main body portion 111, and a second support portion 113-2 for connecting and supporting the other side (for example, a right end) of the base portion 112 and the main body portion 111.

In an embodiment, the base portion 112 as described above may be implemented in a rectangular plate shape having the same or larger size as a lower surface of the main body portion 111. The first support portion 113-1 may be implemented in a rod shape having a length corresponding to one side edge (for example, a left side edge) of the lower surface of the main body portion 111. The second support portion 113-2 may be implemented in a rod shape having a length corresponding to the other side edge (for example, a right side edge) of the lower surface of the main body portion 111.

In addition, in an embodiment, a head-mounted display accommodation portion 114 (hereinafter referred to as an accommodation portion) formed by being surrounded by the lower surface of the main body portion 111, the inner side surface of the support portion 113, and the upper surface of the base portion 112 may be disposed on the body 110.

In detail, in an embodiment, the accommodation portion 114 may be implemented in a hollow (through-type) shape with one side open to provide a space where the head-mounted display 200 of the eye health measurement device may flow in or out.

In addition, the accommodation portion 114 may store the head-mounted display 200 introduced into the accommodation portion 114, and simultaneously, provide a space in which a sterilization/disinfection and/or charging functional operation for the head-mounted display 200 is implemented.

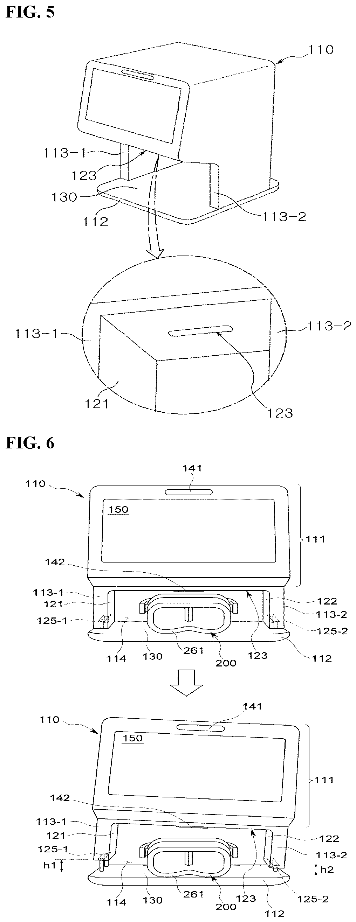

FIGS. 4 and 5 are examples of diagrams for explaining a disinfection portion according to an embodiment of the present invention.

Referring to FIGS. 4 and 5, in an embodiment, the eye health measuring instrument 100 in an embodiment may include the disinfection portion 120 that performs a sterilization and disinfection function for the head-mounted display 200 introduced into the accommodation portion 114.

In detail, in an embodiment, the disinfection portion 120 may perform a functional operation of sterilizing and disinfecting a facial contact protection portion 261 of a housing 260 in the head-mounted display 200 introduced into the accommodation portion 114.

In more detail, the disinfection portion 120 may be implemented with at least one ultraviolet lamp (UV lamp) and/or at least one LED lamp capable of sterilizing and disinfecting an object to be sterilized and disinfected (in an embodiment, the head-mounted display 200).

In addition, according to the control of the eye health measuring instrument 100, the disinfection portion 120 may tilt a lamp group including at least one lamp toward the facial contact protection portion 261 of the head-mounted display 200 introduced into the accommodation portion 114.

In addition, in an embodiment, the disinfection portion 120 may perform an irradiation operation based on lamps tilted toward the facial contact protection portion 261 of the head-mounted display 200.

Thus, the disinfection portion 120 may sterilize and disinfect the facial contact protection portion 261 of the head-mounted display 200 stored in the accommodation portion 114 of the eye health measuring instrument 100.

In detail, in an embodiment, the eye health measuring instrument 100 may acquire position, area, inflow direction and/or angle information of the facial contact protection portion 261 based on the sensor portion 140 capable of sensing the position of the facial contact protection portion 261 of the head-mounted display 200.

In addition, the eye health measuring instrument 100 may set at least one lamp group (for example, a plurality of lamp sets in the third disinfection portion 120 and the second disinfection portion 120) that irradiate the facial contact protection portion 261 based on the position, area, inflow direction and/or angle information of the facial contact protection portion 261 acquired as above.

In addition, the eye health measuring instrument 100 may control to emit light for sterilization and disinfection by operating a set lamp group.

In an embodiment, the disinfection portion 120 may be disposed on an upper side surface of the accommodation portion 114 of the eye health measuring instrument 100.

In other words, in an embodiment, the disinfection portion 120 may include a first disinfection portion 121 disposed inside the first support portion 113-1 forming the accommodation portion 114, a second disinfection portion 122 disposed inside the second support portion 113-2, and a third disinfection portion 123 disposed on a lower surface of the main body portion 111.

In addition, the disinfection portion 120 may adjust the direction of at least one lamp disposed in the first disinfection portion 121, the second disinfection portion 122, and/or the third disinfection portion 123, so as to be irradiated toward the facial contact protection portion 261 of the head-mounted display 200 of the accommodation portion 114.

FIG. 6 is an example of a state illustrating an eye health measurement device including a driving portion according to an embodiment of the present invention.

Referring to FIG. 6, according to an embodiment, the eye health measuring instrument 100 may further include a driving portion 125 capable of assisting a tilting operation of the lamp disposed in the aforementioned disinfection portion 120.

In detail, in an embodiment, the driving portion 125 may be formed between the aforementioned base portion 112 and the support portion 113, and may be implemented in the form of a piston performing an up-and-down motion to be spaced apart from or bonded to the base portion 112 and the support portion 113.

In more detail, the driving portion 125 may include a first driving portion 125-1 disposed between one side of the base portion 112 and the first support portion 113-1 to enable separation or bonding between one side of the base portion 112 and the first support portion 113-1, and a second driving portion 125-2 disposed between the other side of the base portion 112 and the second support portion 113-2 to enable separation and bonding between the other side of the base portion 112 and the second support portion 113-2.

In addition, in an embodiment, the first driving portion 125-1 and/or the second driving portion 125-2 may perform an up-and-down motion (piston operation) under the control of the eye health measuring instrument 100.

Specifically, according to the up-and-down motion of the first driving portion 125-1 under the control of the eye health measuring instrument 100, the first support portion 113-1 connected to the first driving portion 125-1 may also perform an up-and-down motion, and one side of the main body portion 111 supported by the first support portion 113-1 may also perform an up-and-down motion according to the up-and-down motion of the first driving portion 125-1 in a chain.

In the same manner, an up-and-down motion of the second support portion 113-2 may be performed according to the up-and-down motion of the second driving portion 125-2. The other side of the main body portion 111 supported by the second support portion 113-2 may also perform a chain up-and-down motion.

In other words, one side and/or the other side of the main body portion 111 and the support portion 113 disposed on an upper side of the base portion 112 may be spaced apart from or bonded to the base portion 112 according to an up-and-down motion of the first driving portion 125-1 and/or the second driving portion 125-2.

Returning again, in other words, in an embodiment, the eye health measuring instrument 100 may assist a tilting operation of the lamp disposed in the aforementioned disinfection portion 120 through control of the driving portion 125 operating as above.

In detail, the eye health measuring instrument 100 may perform a tilting operation on at least one lamp in the disinfection portion 120 to face the facial contact protection portion 261 of the head-mounted display 200 on the accommodation portion 114.

However, only by tilting the lamp at a fixed position, it may be difficult to implement a high irradiation rate for the facial contact protection portion 261 of the head-mounted display 200 introduced into the accommodation portion 114 at random positions, areas, directions and/or angles.

Accordingly, in an embodiment of the present invention, the eye health measuring instrument 100 implements a tilting operation with respect to the inner side surface of the support portion 113 where the disinfection portion 120 is disposed and the lower surface of the main body portion 111, so that the lamps of the disinfection portion 120 may be tilted in a wider range of angles, positions and/or directions.

In detail, in an embodiment, the eye health measuring instrument 100 may calculate a predetermined irradiation angle such that at least one lamp of the disinfection portion 120 faces the facial contact protection portion 261 of the head-mounted display 200 on the accommodation portion 114.

In addition, the eye health measuring instrument 100 may tilt at least one lamp itself of the disinfection portion 120 so that the calculated irradiation angle is implemented.

In this connection, when the irradiation angle is not satisfied only by tilting the lamp itself, the eye health measuring instrument 100 may control the driving portion 125 to tilt the support portion 113 and the main body portion 111.

In detail, in an embodiment, the eye health measuring instrument 100 may determine a movement height h1 for the first driving portion 125-1 and a movement height h2 for the second driving portion 125-2 based on the irradiation angle.

In addition, the eye health measuring instrument 100 causes the driving portion 125 to perform an up-and-down motion according to the movement height determined as above, thereby performing a tilting operation with respect to the support portion 113 and the main body portion 111.

For example, when the facial contact protection portion 261 is introduced toward the other direction where the second driving portion 125-2 is located, the eye health measuring instrument 100 may set the movement height h1 of the first driving portion 125-1 to be greater than the movement height h2 of the second driving portion 125-2 so that the irradiation operation of the disinfection portion 120 is more smoothly implemented in the other direction, and may position one side of the main body portion 111 and the first support portion 113-1 corresponding to the first driving portion 125-1 higher than the other side of the main body portion 111 and the second support portion 113-2 corresponding to the second driving portion 125-2.

In other words, the eye health measuring instrument 100 may implement a tilting operation through which the support portion 113 and the main body portion 111 are inclined in the other direction.

Thus, the eye health measuring instrument 100 may position the lamps of the disinfection portion 120 to more accurately face the facial contact protection portion 261 on the accommodation portion 114, and thus, the performance of the irradiation for sterilization and disinfection can improve.

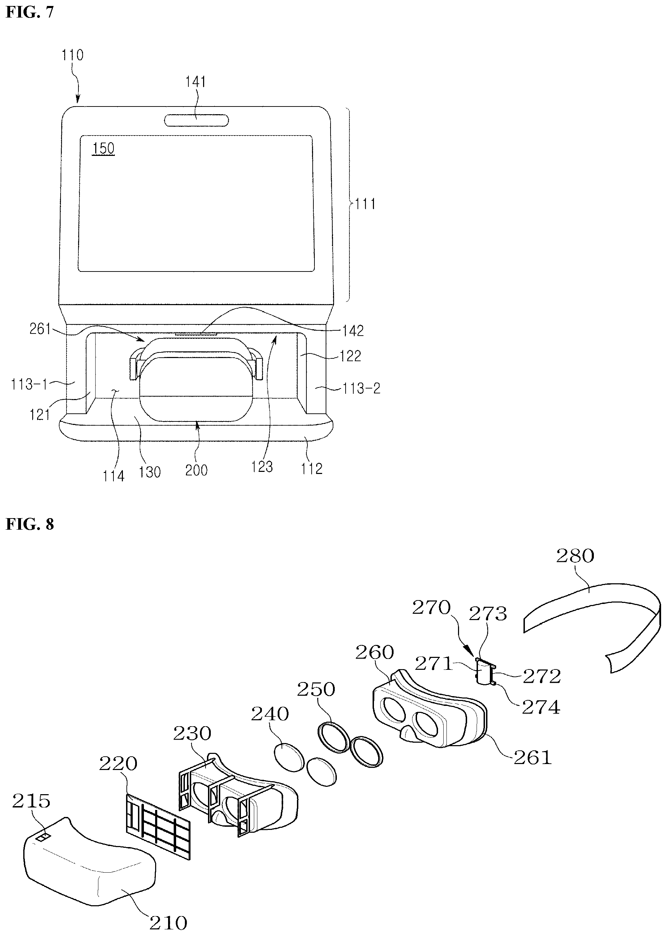

FIG. 7 is an example of a diagram for explaining a charging portion according to an embodiment of the present invention.

Referring to FIG. 7, in an embodiment, the eye health measuring instrument 100 may include the charging portion 130 that provides a charging function for the head-mounted display 200 introduced into the accommodation portion 114.

In detail, in an embodiment, the charging unit 130 may apply a predetermined power to a battery 291 of the head-mounted display 200 seated in the accommodation portion 114 to perform a charging function for the corresponding head-mounted display 200.

In this connection, the eye health measuring instrument 100 may provide a wireless charging and/or wired charging function for the head-mounted display 200.

More specifically, when sensing that the head-mounted display 200 is seated in the accommodation portion 114, the eye health measuring instrument 100 may control the charging portion 130 to automatically execute a wireless charging function for the head-mounted display 200.

In an embodiment, the aforementioned charging portion 130 may be disposed anywhere as long as a charging operation for the head-mounted display 200 disposed in the accommodation portion 114 may be performed. It would be the most preferable embodiment that the charging portion 130 is formed on the upper surface of the base portion 112, which is advantageous for wireless charging, in which performance improves as the charging portion comes into close contact with an object to be charged (in an embodiment, the head-mounted display 200).

In addition, in an embodiment, the eye health measuring instrument 100 may include the sensor portion 140 that senses sensing information required for a functional operation of the eye health measurement device based on various sensing units.

In detail, in an embodiment, the sensor portion 140 may include a user sensing unit 141 that senses the approach of a user (for example, a subject or a measurer) and a head-mounted display sensing unit 142 (hereinafter referred to as a head sensing unit) that senses the introduction of the head-mounted display 200 into the accommodation portion 114.

In more detail, the user sensing unit 141 may acquire user proximity information by determining whether a user approaches close to the eye health measuring instrument 100 by a predetermined distance or less.

In this connection, in an embodiment, the eye health measuring instrument 100 may automatically execute an eye health measurement service when it is determined that a user exists in a position close to the eye health measuring instrument 100 by a predetermined distance or less based on the user proximity information acquired based on the aforementioned user sensing portion 141.

In an embodiment, the eye health measuring instrument 100 may output a graphic image related to the eye health measurement service through the display portion 150 when a user approaches the eye health measuring instrument 100 by a predetermined distance or less.

In addition, in an embodiment, the eye health measuring instrument 100 may output audio data for inducing use of the eye health measurement service using the eye health measuring instrument 100 when a user approaches the eye health measuring instrument 100 by a predetermined distance or less.

In an embodiment, the aforementioned user sensing unit 141 may be implemented as a proximity sensor, a distance sensor, and/or an image sensor that senses the approach of a specific object.

In addition, the head sensing unit 142 may acquire head-mounted display 200 accommodation information (hereinafter referred to as head accommodation information) by determining whether the head-mounted display 200 is accommodated in the eye health measuring instrument 100.

In an embodiment, the head sensing unit 142 may include a proximity sensor, a distance sensor, and/or an image sensor.

In detail, in an embodiment, the head sensing unit 142 may determine whether the head-mounted display 200 is flowed in or flowed out of the accommodation portion 114 of the eye health measuring instrument 100 through sensing.

In addition, in an embodiment, when it is determined that the head-mounted display 200 is introduced into the accommodation portion 114 based on the head accommodation information acquired through the head sensing unit 142, the eye health measuring instrument 100 may sense and track the position of the facial contact protection portion 261 of the head-mounted display 200.

In this connection, in an embodiment, the eye health measuring instrument 100 may perform a tilting operation based on the aforementioned disinfection portion 120 and/or driving portion 125 based on the sensed position information of the facial contact protection portion 261.

In addition, in an embodiment, the eye health measuring instrument 100 may control the disinfection portion 120 tilted toward the facial contact protection portion 261 to perform irradiation based on a UV lamp and/or an LED lamp, through which a sterilization and disinfection function for the facial contact protection portion 261 may be implemented.

In addition, in an embodiment, when it is determined that the head-mounted display 200 is introduced into the accommodation portion 114 based on the head accommodation information acquired through the head sensing unit 142, the eye health measuring instrument 100 may control the aforementioned charging portion 130 to perform an automatic charging function for the head-mounted display 200.

In an embodiment, the sensor portion 140 may be disposed anywhere as long as it is easy to sense the head-mounted display 200 flowing in or flowing out of the eye health measuring instrument 100. However, it may be the most preferable embodiment that the sensor portion 140 is disposed on the accommodation portion 114, which is a region where the head-mounted display 200 is directly seated.

In an embodiment, the eye health measuring instrument 100 may include the display portion 150 that outputs various graphic images related to the eye health measurement service.

In detail, in an embodiment, the display portion 150 may output a user interface related to the eye health measurement service, an eye health condition measurement result, and/or a related graphic image for controlling the head-mounted display 200.

In an embodiment, the eye health measuring instrument 100 may control the display portion 150 to output sterilization/disinfection information for the facial contact protection portion 261 of the head-mounted display 200.

Herein, the sterilization and disinfection information according to an embodiment may be information obtained by calculating a degree of sterilization of the facial contact protection portion 261 of the head-mounted display 200 by the disinfection portion 120 according to a predetermined criterion.

For example, the eye health measuring instrument 100 may generate a graphic image based on sterilization/disinfection information that increases a sterilization/disinfection progress rate whenever a predetermined time slice elapses based on the lamp emission time of the disinfection portion 120 and output the same through the display portion 150.

In addition, according to an embodiment, the display portion 150 may further include an audio output portion to provide audio data related to the output graphic image.

The display portion 150 may include at least one of a liquid crystal display (LCD), a thin film transistor liquid crystal display (TFT LCD), an organic light emitting diode (OLED), a flexible display, and an e-ink display.

In addition, in an embodiment, the eye health measuring instrument 100 may include the input portion 160 that senses a user input related to the eye health measurement service.

For example, the input portion 160 may sense a user input for an eye health measurement service execution button, a measurement category selection button, and/or a subject information input interface.

In addition, according to an embodiment, the aforementioned display portion 150 and the input portion 160 may be combined to be implemented as a touch screen 161.

In other words, the display portion 150 of the eye health measuring instrument 100 may provide an input/output interface for sensing a touch input of a user by further disposing a touch input sensor on a display panel that outputs a graphic image.

Referring further to FIG. 2, in an embodiment of the present invention, the eye health measuring instrument 100 may further include built-in units such as an interface portion 171, a communication portion 172, a database portion 173, a power supply portion 174, and a processor 175.

In detail, in an embodiment, the interface portion 171 may be a data passage for data communication between an external device (in an embodiment, the head-mounted display 200) and the eye health measuring instrument 100.

In an embodiment, the interface portion 171 may be wired connected to an external device through various ports and/or cables, and may perform data communication with the external device through a short-range wireless communication module such as Bluetooth or Wi-Fi.

The interface portion 171 may include at least one of wired or wireless headset ports, external power supply ports, wired or wireless data ports, memory card ports, ports for connecting a device having an identification module, audio input/output (I/O) ports, video I/O ports, and/or earphone ports.

In addition, in an embodiment, the communication portion 172 may transmit/receive various pieces of data related to the eye health measurement service with an external device (in an embodiment, the head-mounted display 200).

In this connection, the communication portion 172 may perform the data transmission and reception based on a wireless network.

The communication portion 172 may transmit/receive wireless signals to/from at least one of network entities, for example, a base station, an external mobile terminal, a server, and the like, on a mobile communication network, which is constructed according to technical standards or communication methods for mobile communications (for example, Global System for Mobile communication (GSM), Code Division Multi Access (CDMA), High Speed Downlink Packet Access (HSDPA), High Speed Uplink Packet Access (HSUPA), Long Term Evolution (LTE), Long Term Evolution-Advanced (LTE-A), etc.).

In addition, the communication portion 172 may also transmit/receive wireless signals using a wireless communication method such as Wireless LAN (WLAN), Wireless-Fidelity (Wi-Fi), Wireless Fidelity Direct (Wi-Fi Direct), Digital Living Network Alliance (DLNA), Wireless Broadband (WiBro), World Interoperability for Microwave Access (WiMAX), and the like.

In addition, the communication portion 172 may transmit/receive wireless signals using a short-range wireless communication method. For example, the communication portion 172 may support short-range communication using at least one of BLUETOOTH^{™}, Radio Frequency IDentification (RFID), Infrared Data Association (IrDA), Ultra-WideBand (UWB), ZigBee, Near Field Communication (NFC), Wireless-Fidelity (Wi-Fi), Wi-Fi Direct, Wireless Universal Serial Bus (Wireless USB) and the like.

In addition, in an embodiment, the database portion 173 may store and manage various application programs, applications, instructions, and/or data for implementing the eye health measurement service.

In an embodiment, this database portion 173 may include a program area and a data area.

Herein, the program area according to an embodiment may be linked between an Operating System (OS) and functional elements for booting the eye health measuring instrument 100, and the data area may store data generated according to the use of the eye health measuring instrument 100.

In addition, in an embodiment, the database portion 173 may be various storage devices such as a ROM, a RAM, an EPROM, a flash driver, and a hard drive, and may be a web storage that performs the storage function of the database portion 173 on the Internet.

In addition, in an embodiment, the power supply portion 174 may receive external power and/or internal power under the control of a processor 175 to supply power required for operation to each component.

For example, the power supply portion 174 may include at least one of a power storage portion, a connection port, a power supply controller, and a charge monitoring portion.

In addition, in an embodiment, the processor 175 may control and drive the overall operation of each unit described above.

In other words, the processor 175 may control the overall operation of each unit for the eye health measurement service.

In addition, according to an embodiment, one or more processors 175 may be connected through the interface portion 171 to drive or execute various software programs and/or sets of instructions stored in the database portion 173 to perform various functions of the eye health measuring instrument 100 and process data.

The processor 175 may be implemented using at least one of application specific integrated circuits (ASICs), digital signal processors (DSPs), digital signal processing devices (DSPDs), programmable logic devices (PLDs), field programmable gate arrays (FPGAs), controllers, micro-controllers, microprocessors, and electronic units for executing other functions.

### <Head-mounted Display 200>

Next, the head-mounted display 200 according to an embodiment is a device that is worn on a subject like wearing a helmet or eyeglasses and then can quickly and accurately measure eye health conditions of the subject using an optical unit and a display unit disposed in the head-mounted display 200.

In detail, the head-mounted display 200 according to an embodiment may acquire and output virtual reality (VR) images related to eye health condition measurement from the eye health measuring instrument 100 and may generate reaction of subject's eyes to the output VR images or/and input from the subject as measurement data.

In addition, the head-mounted display 200 may transmit and process the generated measurement data directly or to the eye health measuring instrument 100, and the transmitted measurement data may be used to derive an eye health condition measurement result. In other words, the eye health condition measurement result may be acquired based on measurement data.

According to the head-mounted display 200, a subject may take a measurement while freely moving or at a comfortable position without taking the measurement in a state fixed to predetermined equipment (in an embodiment, the eye health measuring instrument 100) for measuring eye health conditions of the subject in a measurement of the eye health conditions.

In addition, the head-mounted display 200 of an embodiment of the present invention implements the measurement of eye health conditions of a subject in the form of a virtual reality (VR) device, so that the eye health conditions of the subject may be measured based on a virtual reality image without a separate analog measuring device or expensive equipment (for example, perimetry).

In addition, the head-mounted display 200 of an embodiment of the present invention allows the eye health conditions of a subject to be progressed in a normalized automatic measurement type, thus extracting objective measurement data and determining accurate eye health conditions based thereon.

For example, the head-mounted display 200 may be implemented as a wearable computer or may have the type of a wearable computer (or referred to as a wearable computing device). In an exemplary embodiment, the wearable computer may be a head-mountable display (HMD) or may include an HMD. The head-mounted display 200 may be any device that may be worn on the head and may show a display in front of one or both eyes of a wearer. The HMD may have various types such as a helmet or eyeglasses.

FIG. 8 is an exploded perspective view of a head-mounted display according to an embodiment of the present invention.

Referring to FIG. 8, the head-mounted display 200 according to an embodiment may include a main body 210, and a display unit 220 sequentially accommodated on the rear surface of the main body 210, an eye examination unit 230, an optical unit 240, an optical holder 250, the housing 260, an anti-optical interference unit 270, and a fixing band 170.

However, the components shown in FIG. 8 are not necessary components of the head-mounted display 200, so they may not be provided, depending on embodiments. Herein, the rear surface of the main body 2100 means the direction in which an opening is formed and the face of a subject touches and the front surface of the main body 210 means the gaze direction of a subject.

First, the main body 210 may be formed in a plastic and/or metallic solid structure or may be formed in a hollow structure made of similar materials such that wires and components are connected to each other to be internally routed through the head-mounted display 200.

Reference numeral '215' shown in the figures indicates an input unit 215. The input unit 215 may turn on/off the head-mounted display 200 or may sense input related to an eye health condition measurement.

In detail, the input unit 120 may sense user (a subject or a measurer) input for performing communication between the head-mounted display 200 and an external system or may sense input for changing and selecting measurement kinds when a user measures eye health conditions.

Further, depending on cases, an alarm type LED light may be further included to make it possible to recognize the progress state of a measurement or the state after measurement is finished from the outside.

Further, the head-mounted display 200 may further include an external input unit wired/wirelessly connected with the head-mounted display 200 and the external input unit may be a device that senses input for deriving the intention of a measurer according to eye health condition measurement progress.

The external input unit records the point in time of input and an input value by a measurer and transmits the recorded input value to the head-mounted display 200 or the eye health measuring instrument 100 and the transmitted input value may be included in the measurement data.

Next, a fixing band 280 is shown as two pieces of fixing band portions, but is not limited thereto.

In addition, according to an embodiment, the fixing band 280 may provide a helmet type to be able to fix the main body 210 to the face of a subject or three or more bands to be able to fix the main body 210 while surrounding the head of a subject.

Next, the display unit 220 may include at least one of a liquid crystal display (LCD), a thin film transistor liquid crystal display (TFT LCD), an organic light emitting diode (OLED), a flexible display, and an e-ink display.

The display unit 220 may finally display graphic images as 3-dimensional display to measure eye health conditions.

For example, the display unit 220 may display VR images of 3-dimensional display and uses the same for measuring eye health conditions.

Further, when the panel of the display unit 220 is provided as a single display panel, separate images corresponding to the left eye and the right eye of a subject respectively may be implemented. Depending on cases, the panel may be configured of at least two separated display panels.

Next, the eye examination unit 230 may include a plurality of sensors, a plurality of cameras, and a controller of the head-mounted display 200 and a circuit module that may communicate with external systems to be able to measure various eye health conditions of subj ects.

The eye examination unit 230 may provide an eye tracking function that tracks the eyes of a subject who takes an eye measurement. To this end, cameras that may track motion of pupils of a subject may be mounted in the eye examination unit 230.

In addition, the eye examination unit 230 may acquire measurement data that are the base for measuring eye health conditions such as visual field measurement, strabismus angle measurement, extrinsic ocular muscle measurement, stereopsis measurement, or Lancaster measurement by tracking the gaze of a subject that changes dependent on VR images.

In detail, an eyeball image of a subject captured by the eye examination unit 230 may be included in the measurement data and transmitted to the eye health measuring instrument 100 and the transmitted measurement data may be the base of the measurement result.

In other words, a light radiation member and a photographing member that are required for visual field measurement, strabismus angle measurement, extrinsic ocular muscle measurement, stereopsis measurement, or Lancaster measurement performed for measuring eye health conditions may be implemented in the type of a sensor module or a camera module.

Further, the eye examination unit 230 may further include a sensor that may acquire refractive index measurement information about the eyes of a subject by radiating light to the left eye and the right eye of the subject and receiving light for measuring eye health conditions.

Next, the optical unit 240 is positioned between the eye examination unit 230 and the housing 160 and may provide the most suitable optical unit 240 corresponding to the kind of an eye health condition measurement of a subject.

For example, when the eye health condition measurement of a subject is eyesight measurement, optical unit 240 may selectively replace and fasten an optical lens for measuring the refractive index of crystalline lenses.

Further, in an embodiment, when the ophthalmic measurement of a subject is motion measurement of pupils such as an extrinsic ocular muscle measurement, the optical unit 240 may be an optical lens that makes it possible to precisely see motion of pupils through a camera.

In addition, in an embodiment, the optical unit 240 may be an optical unit having a structure in which several lenses having a polarization characteristic are stacked.

The optical unit 240 is fixed to the optical holder 250. Necessary optical units 240 may be replaced and detachably attached to the optical holder 250, depending on the kinds of eye health condition measurements.

Next, the housing 260 is inserted in the direction of the opening of the main body 210 and fixes the display unit 220, the eye examination unit 230, the optical unit 240, the optical holder 250, or/and the main body 210 while maintaining optical alignment.

Further, it provides a space between the display and both eyes of a subject in which an eye health condition measurement may be performed when the subject wears the head-mounted display 200.

Accordingly, the inside of the housing 260 may include a left eye region and a right eye region that is independently separated by the anti-optical interference unit 270.

Further, the inner surface of the housing 260 may be coated with a material having low light reflection and a high light absorption ratio for eye health condition measurements.

In addition, in an embodiment, the housing 260 may dispose the facial contact protection portion 261 on an opening side of the housing 260.

In detail, in an embodiment, the facial contact protection portion 261 may be formed at a portion where the head-mounted display 200 and the facial skin of a subject contact (in other words, the region on the opening side of the housing 260), and may perform a fixing and shock absorption action between the head-mounted display 200 and the face of the subject.

In an embodiment, the facial contact protection portion 261 may receive wavelengths (light) emitted from an ultraviolet lamp (UV lamp) and/or an LED lamp disposed in the disinfection portion 120 of the eye health measuring instrument 100, and thus, sterilization and disinfection treatment may be implemented.

Since the facial contact protection portion 261 is a part that is supposed to come in contact with the facial skin of a subject, it is preferable that the facial contact protection portion 261 is made of a material having a high shock absorption ability and is cleanly protected from any contamination.

Next, the anti-optical interference unit 270 may separate the head-mounted display 200 into a left eye region and a right eye region for measuring eye health conditions of a subject. In general, people have two eyes with similar shapes at similar positions, but each eye independently functions, so eye health conditions also show similar but slightly different symptoms. Accordingly, there are measurements for independently performing eye health condition measurements for the left eye and the right eye.

However, when light that is used during an eye health condition measurement for the left eye influences the adjacent right eye or vice versa, it is difficult to accurately and precisely measure eye health conditions.

For example, when only the left eye is measured with the right eye covered or vice versa, if the light output from a region interferes with the other regions, it may have a bad influence on the measurement result.

Accordingly, the head-mounted display 200 according to an embodiment includes the anti-optical interference unit 270, thereby being able to block light generated in the left eye region and the right eye region from invading regions each other.

The anti-optical interference unit 270 may include an optical block portion 271 that separates the head-mounted display 200 into the left eye region and the right eye region and blocks the light traveling to an adjacent region from each region.

In order to further increase the optical block ratio, the anti-optical interference unit 270 may include a contact-optical block portion 272 for preventing optical interference that is generated at the optical block portion 271 and the region (brow region) between the left eye and right eye regions of a subject when the subject wears the head-mounted display 200, and may further include a first fixing portion 273 and a second fixing portion 274 that fix and support the anti-optical interference unit 270 in the brow region and the nose ridge region of the subject.

Accordingly, the optical block portion 271 is not simply supported by the nose of a wearer and has a shape corresponding to the shape between the brow and the philtrum, so it is possible to prevent light leakage between the left eye region and the right eye region by separating the left eye region and the right eye region when the head-mounted display 200 is worn.

Further, it is preferable that the optical block portion 271 and the contact-optical block portion 272 are made of a material having small light reflection and a high light absorption ratio.

Further, since the contact-optical block portion 272 is a part that is supposed to come in contact with the face skin of a subject, it is preferable that the contact-optical block 302 is made of a material having a high shock absorption ability.

Since the left eye region and the right eye region are optically separated in the region for measuring eye health conditions in the head-mounted display 200, there is a benefit that it is possible to prevent examination errors due to optical interference that may be generated during measurement.

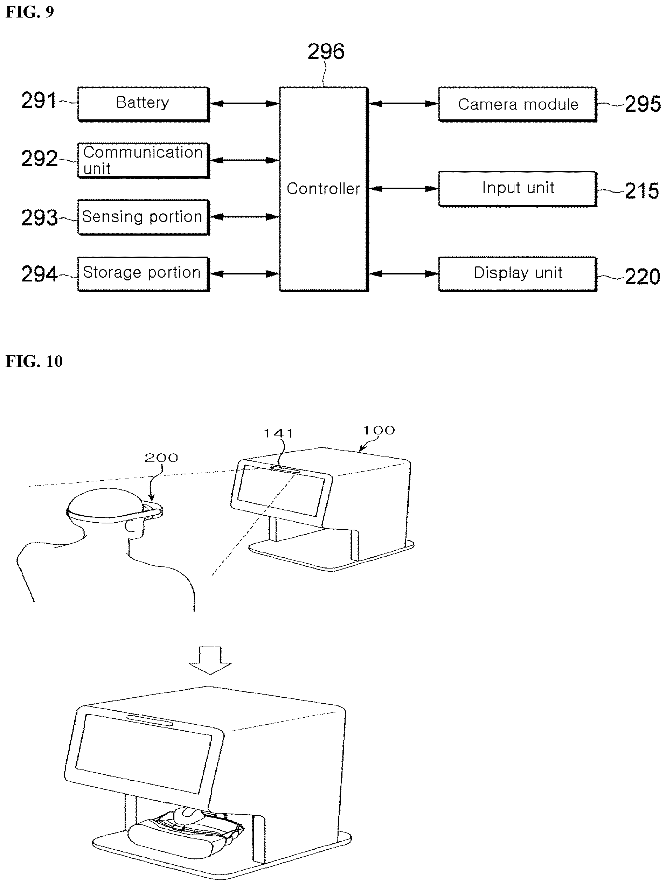

FIG. 9 is an internal block diagram of a head-mounted display according to an embodiment of the present invention.

Further, referring to FIG. 9, in terms of function, the head-mounted display 200 according to an embodiment may include the battery 291, a communication unit 292, a sensing portion 293, a storage portion 294, a camera module 295, an input unit 251, the display unit 220, and a controller 296.

However, the components shown in FIG. 9 are also not necessary parts, so the heatmounted display 200 may be composed of more or less components. Hereafter, the components are sequentially described.

First, the controller 296 usually controls the general operation of the head-mounted display 200. For example, it is possible to transmit/receive various signals or process input data by controlling the communication unit 292.

Further, it can provide information to a subject or a measurer by controlling an image output unit and/or a sound output unit that may be disposed in the display unit 220.

Depending on embodiments, the head-mounted display 200 may extract measurement data obtained by measuring the eyes of a subject, may perform comparison and direct calculation on the basis of the eye health condition data stored in the storage portion 294, and then may determine the eye health condition of the subject.

In addition, the battery 291 is controlled by the controller 296 to be provided with external power and internal power and to supply power for the operation of each component.

For example, the battery 291 may include a battery, a connection port, a power supply controller, and a charge monitoring unit.

In addition, the camera module 295 processes image frames such as still images or moving images captured by an image sensor in a video call mode or a photographing mode. The processed image frames can be stored in the storage portion 294 or transmitted to an external system through the communication unit 292.

At least two or more camera modules 295 may be provided, depending on the measurement kind or measurement environment of the eye health conditions of a subject.

For example, when a subject takes eyesight measurement, a camera may be used to monitor the pupils. Further, when the motion of eyeballs of a subject is tracked, a camera that may take photographs along the movement path of the eyeballs may be used.

In addition, the communication unit 292 enables wire/wireless communication with the eye health measuring instrument 100 that is an external system. Herein, the external system may be a concept including another head-mounted display 200.

The communication unit 190 may include a mobile communication module, a wireless internet module, a short range communication module, and a position information module.

The sensing portion 293 may include a gyro sensor that senses the surrounding environment, an acceleration sensor, and/or a proximity sensor.

In particular, the head-mounted display 200 of an embodiment of the present invention may include at least two or more optical sensors that generate light to be radiated to the eyes of a subject and receive light to measure eye health conditions.

In addition, the storage portion 294 may keep applications for processing and controlling by the controller 296 and may also temporarily store input/output data.

### - Method of Operating Eye Health Measurement Device

Hereinafter, a method of operating an eye health measuring device according to an embodiment of the present invention will be described in detail with reference to FIG. 9.

FIG. 10 is an example of a diagram for explaining the operating principle of the eye health measurement device according to an embodiment of the present invention.

Referring to FIG. 10, in an embodiment, the eye health measuring instrument 100 of the eye health measurement device may sense proximity of a subject whose eye health condition is to be measured based on the user sensing unit 141 of the sensor portion 140.

In addition, the eye health measuring instrument 100 may automatically execute the eye health measurement service when sensing that a subject approaches by a predetermined distance or less.

In this connection, according to an embodiment, the eye health measuring instrument 100 may output a graphic image informing that the eye health measurement service is executed through the display portion 150 or output predetermined audio data in conjunction with the audio output portion of the display portion 150 and/or an external audio device connected to the interface portion 171.

In addition, in an embodiment, in order to proceed with the eye health measurement service, the eye health measuring instrument 100 may sense leakage of the head-mounted display 200 stored in the accommodation portion 114 by a user (in an embodiment, a subject and/or measurer).

In detail, the eye health measuring instrument 100 may sense outflow of the head-mounted display 200 in the accommodation portion 114 based on the head sensing unit 142 of the sensor portion 140.

Thereafter, the eye health measuring instrument 100 may sense the head-mounted display re-introduced into the accommodation portion 114 through the head sensing unit 142 of the sensing portion as an eye health condition measurement process based on the head-mounted display 200 and the eye health measuring instrument 100 is completed.

In this connection, in an embodiment, when the head-mounted display 200 is introduced into the accommodation portion 114, the eye health measuring instrument 100 may sense and track the position of the facial contact protection portion 261 of the introduced head-mounted display 200.

In detail, in an embodiment, the eye health measuring instrument 100 may acquire position, area, inflow direction and/or angle information of the facial contact protection portion 261 (hereinafter referred to as protection portion sensing information) of the head-mounted display 200 introduced into the accommodation portion 114 at random positions, areas, directions and/or angles based on the head sensing unit 142.

In addition, the eye health measuring instrument 100 may control the disinfection portion 120 based on the acquired protection portion sensing information to set the light irradiation direction of at least one lamp group in the disinfection portion 120.

Specifically, in an embodiment, the eye health measuring instrument 100 may determine a lamp group including at least one lamp among a plurality of lamps in the disinfection portion 120 based on the protection portion sensing information.

For example, the eye health measuring instrument 100 may determine a lamp group including at least one lamp disposed on a region facing the facial contact protection portion 261 based on the protection portion sensing information.

In addition, in an embodiment, the eye health measuring instrument 100 may perform a predetermined tilting operation with respect to the determined lamp group.

In an embodiment, the eye health measuring instrument 100 may tilt each of the lamp groups by a predetermined angle toward the facial contact protection portion 261.

In other words, in an embodiment, the eye health measuring instrument 100 may adjust the irradiation angle of a lamp emitting ultraviolet light and/or LED for sterilization and disinfection to be optimized for the direction toward the facial contact protection portion 261 of the head-mounted display 200, thereby improving accuracy and effectiveness of sterilization and disinfection treatment for the facial contact protection portion 261 of the head-mounted display 200 seated in the accommodation portion 114 with a random position, area, direction and/or angle.

In this connection, according to an embodiment, when the irradiation rate of the facial contact protection portion 261 does not meet a predetermined criterion only by the tilting operation of the disinfection portion 120, the eye health measuring instrument 100 implements a tilting operation with respect to an inner side surface of the support portion 113 where the disinfection portion 120 is disposed and a lower surface of the main body portion 111, so that the lamps of the disinfection portion 120 may be tilted in a wider range of angles, positions, and/or directions.

In detail, in an embodiment, when the irradiation angle is not satisfied only by tilting the disinfection portion 120 (for example, a lamp) itself, the eye health measuring instrument 100 may control the driving portion 125 to implement a tilting operation with respect to the support portion 113 and the main body portion 111.

More specifically, in an embodiment, the eye health measuring instrument 100 may determine a movement height h1 for the first driving portion 125-1 and a movement height h2 for the second driving portion 125-2 based on the irradiation angle.

In addition, the eye health measuring instrument 100 causes the driving portion 125 to perform an up-and-down motion according to the movement height determined as above, thereby performing a tilting operation with respect to the support portion 113 and the main body portion 111.

For example, when the facial contact protection portion 261 is introduced toward the other direction where the second driving portion 125-2 is located, the eye health measuring instrument 100 may set the movement height h1 of the first driving portion 125-1 to be greater than the movement height h2 of the second driving portion 125-2 so that the irradiation operation of the disinfection portion 120 is more smoothly implemented in the other direction, and may position one side of the main body portion 111 and the first support portion 113-1 corresponding to the first driving portion 125-1 higher than the other side of the main body portion 111 and the second support portion 113-2 corresponding to the second driving portion 125-2.

In other words, the eye health measuring instrument 100 may implement a tilting operation through which the support portion 113 and the main body portion 111 are inclined in the other direction.

Thus, the eye health measuring instrument 100 may position the lamps of the disinfection portion 120 to more accurately face the facial contact protection portion 261 on the accommodation portion 114, and thus, the performance of the irradiation for sterilization and disinfection can improve.

In addition, in an embodiment, the eye health measuring instrument 100 may generate sterilization and disinfection information, which is information obtained by calculating a degree of sterilization of the facial contact protection portion 261 based on the disinfection portion 120 according to a predetermined criterion.

In addition, the eye health measuring instrument 100 may control the display portion 150 to output the generated sterilization and disinfection information, and provide the same to a user.

For example, the eye health measuring instrument 100 may generate a graphic image based on sterilization/disinfection information that increases a sterilization/disinfection progress rate whenever a predetermined time slice elapses based on the lamp emission time of the disinfection portion 120 and output the same through the display portion 150.

In an embodiment, when the head-mounted display 200 is introduced into the accommodation portion 114, the eye health measuring instrument 100 may charge the head-mounted display 200 by controlling the charging portion 130.

In detail, in an embodiment, when sensing that the head-mounted display 200 is seated in the accommodation portion 114, the eye health measuring instrument 100 may control the charging portion 130 to automatically execute a wireless charging function for the head-mounted display 200.

In addition, in an embodiment, the eye health measuring instrument 100 may acquire charging information (for example, charging rate (%) and/or charging elapsed time information) calculated as the charging progresses.

In addition, the eye health measuring instrument 100 may graphically image the acquired charging information and output the same through the display portion 150.

For example, the eye health measuring instrument 100 may output, through the display portion 150, the elapsed charging time for the head-mounted display 200, the amount of charge charged during the corresponding time, and the charge rate information as a graphic image.

As such, the eye health measurement device according to an embodiment of the present invention provides a functional operation (in an embodiment, a sterilization/disinfection function) to keep the head-mounted display used for eye health measurement clean. Accordingly, it is possible to safely maintain the cleanliness of the head-mounted display used for an eye health measurement service without checking a contamination state of the head-mounted display every time or manually performing a separate sterilization treatment.

In addition, the virtual reality-based eye health measurement device provides a functional operation (in an embodiment, an automatic charging function) that assists in convenient management of the head-mounted display used for eye health measurement. Accordingly, it is possible to easily and conveniently manage eye health condition measurement environment.

### - 2) Eye Health Platform Management Server

The eye health platform management server 300 according to an embodiment of the present invention may perform a series of processes for providing an eye health measurement service.

In detail, in an embodiment, the eye health platform management server 300 may provide the eye health measurement service by exchanging data necessary for driving an eye health condition measurement process in an external device such as the eye health measuring instrument 100 and/or the head-mounted display 200 with the external device.

In more detail, in an embodiment, the eye health platform management server 300 may provide an environment in which an eye health condition measurement process may operate in an external device (for example, the eye health measuring instrument 100, the head-mounted display 200, and/or a mobile type computing device).

In addition, the eye health platform management server 300 may provide an eye health condition survey process for performing predetermined questions and answers to determine the eye health condition of a subject.

In addition, the eye health platform management server 300 may determine an eye health measurement method optimized for a corresponding subject based on the eye health condition survey process.

In addition, the eye health platform management server 300 may provide a virtual reality-based eye health condition measurement process based on the determined eye health measurement method.

In addition, the eye health platform management server 300 may provide result information according to eye health condition measurement based on various routes.

In addition, the eye health platform management server 300 may store and manage various pieces of data related to the eye health measurement service.

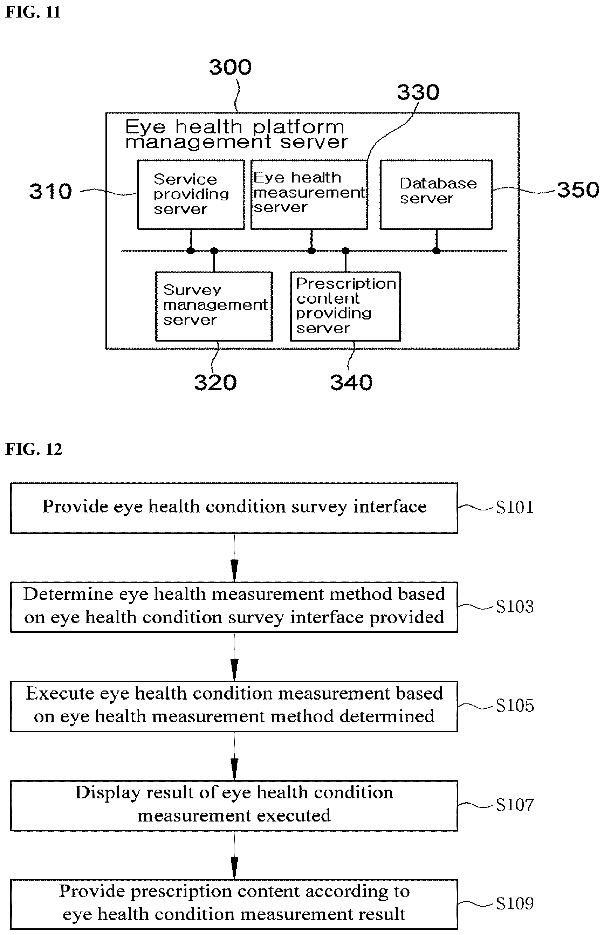

FIG. 11 is an internal block diagram of an eye health platform management server according to an embodiment of the present invention.

In more detail, referring to FIG. 11, in an embodiment, the eye health platform management server 300 may include a service providing server 310, a survey management server 320, an eye health measurement server 330, a prescription content providing server 340, and a database server 350.

In this connection, according to an embodiment, each of the components may be implemented as a device separate from the eye health platform management server 300 or may be implemented by being included in the eye health platform management server 300. Hereinafter, each component will be described as being implemented by being included in the eye health platform management server 300, but is not limited thereto.

In detail, in an embodiment, the service providing server 310 may provide an environment in which an eye health condition measurement process may operate in an external device (for example, the eye health measuring instrument 100, the head-mounted display 200, and/or a mobile type computing device).

In other words, the service providing server 310 may provide an environment in which an eye health condition measurement process providing an eye health measurement service may be executed in an external device.

To this end, the service providing server 310 may include an application program, data, and/or instructions for implementing the eye health condition measurement process.

In addition, in an embodiment, a survey management server 320 may provide an eye health condition survey process for performing a predetermined question and answer to determine the eye health condition of a subject.

In an embodiment, the survey management server 320 may provide an eye health condition survey interface capable of performing an eye health condition survey process.

In addition, the survey management server 320 may provide predetermined query items related to the eye health condition based on the provided survey interface.

In addition, the survey management server 320 may acquire a response of a user (in an embodiment, a subject or measurer) to the provided query item based on a user input based on the survey interface.

In addition, in an embodiment, the eye health measurement server 330 may provide at least one eye health condition measurement process.

In detail, in an embodiment, the eye health measurement server 330 may manage and provide an eye health condition measurement process that implements eyesight measurement, visual field measurement, astigmatism measurement, macular degeneration measurement, color blindness measurement, stereopsis measurement, diplopia measurement, contrast sensitivity measurement, extrinsic ocular muscle measurement and/or dynamic eyesight measurement.

In addition, in an embodiment, the prescription content providing server 340 may provide prescription content (in an embodiment, eye healing images and/or information on recommended nutritional supplements) according to the eye health condition measurement result.

Herein, the prescription content according to an embodiment may be content data provided for the purpose of improving an eye health condition of a subject based on the measurement result of the eye health condition.

In addition, in an embodiment, the database server 350 may store and manage various application programs, applications, instructions, and/or data for implementing the eye health measurement service.

In an embodiment, the database server 350 may store and manage eye health condition questionnaire information, score information given for each query item in the questionnaire, eye health condition survey information, eye health measurement method information (in an embodiment, stereopsis measurement content information and/or visual field content information), eye health condition information, and/or prescription content information.

The eye health platform management server 300 including the aforementioned components may be configured of at least one service providing server 310, the survey management server 320, the eye health measurement server 330, the prescription content providing server 340 and/or the database server 350, and may include processors for data processing and memories for storing instructions for providing an eye health measurement service.

In addition, hereinbefore, it has been described that the eye health platform management server 300 may provide an environment in which an eye health condition measurement process may operate in an external device, provide an eye health condition survey process, determine an eye health measurement method optimized for a subject based on the provided eye health condition survey process, provide a virtual reality-based eye health condition measurement process based on the determined eye health measurement method, provide result information according to eye health condition measurement, and store and manage various pieces of data related to the eye health measurement service. However, according to an embodiment, various embodiments may also be possible, such as that at least some of the functional operations performed by the eye health platform management server 300 may also be performed in an external device (for example, the eye health measuring instrument 100 and/or the head-mounted display 200).

### - Virtual Reality-Based Eye Health Measurement Method

Hereinafter, a method for measuring eye health based on virtual reality by the eye health measurement system according to an embodiment of the present invention will be described in detail with reference to the accompanying drawings.

The eye health measurement system according to an embodiment of the present invention may be implemented based on at least some of the aforementioned eye health measuring instrument 100, the head-mounted display 200, and the eye health platform management server 300.

In detail, the eye health measurement system according to an embodiment may be implemented based on an eye health measurement device, that is, the eye health measuring instrument 100 and the head-mounted display 200. (First system mode)

In detail, in an embodiment, the eye health measurement system may provide an eye health measurement service based on the eye health measuring instrument 100 implementing the eye health measurement service and the head-mounted display 200 implementing a virtual reality environment related to the eye health measurement service.

In another embodiment, the eye health measurement system may be implemented based on the head-mounted display 200 and the eye health platform management server 300.

### (Second system mode)

In detail, in another embodiment, the eye health measurement system may provide an environment in which the eye health measurement service may operate in the head-mounted display 200 based on the eye health platform management server 300, and provide the eye health measurement service based on the head-mounted display 200 provided with this operating environment.

In yet another embodiment, the eye health measurement system may be implemented based on the eye health measuring instrument 100 and a web server. (Third system mode)

In detail, in yet another embodiment, the eye health measurement system may provide an eye health measurement service online in conjunction with the eye health measuring instrument 100 with an external web server.

The virtual reality-based eye health measurement system according to an embodiment of the present invention may be implemented in any of the three system modes described above, but hereinafter, for effective description, description will be made based on the first system mode implemented based on the eye health measuring instrument 100 and the head-mounted display 200 (in other words, an eye health measurement device).

Hereinafter, a method in which the processor 175 of the eye health measuring instrument 100 according to an embodiment of the present invention is linked with the head-mounted display 200 to measure eye health conditions based on virtual reality will be described in detail with reference to the accompanying drawings.

FIG. 12 is a flowchart illustrating a method for virtual reality-based eye health measurement according to an embodiment of the present invention.

Referring to FIG. 12, the processor 175 of the eye health measuring instrument 100 according to an embodiment of the present invention may provide the eye health condition survey interface (S101)

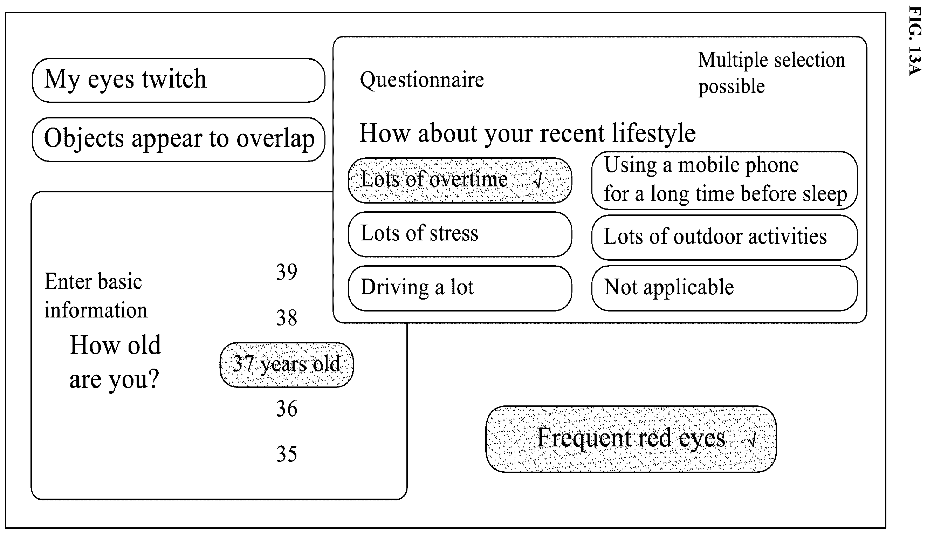

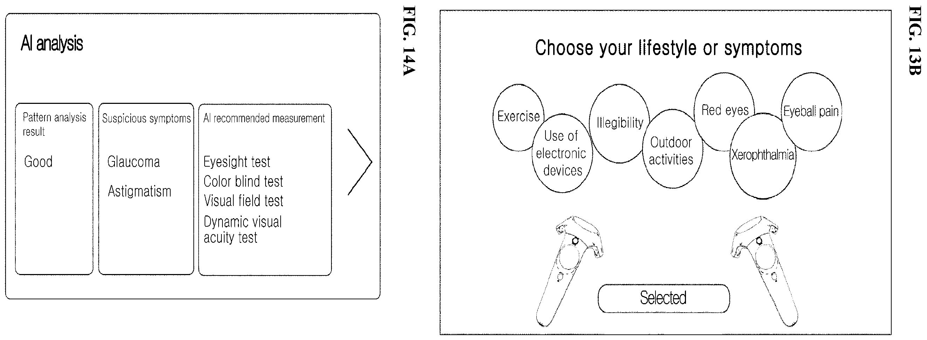

FIGS. 13A and 13B are examples of an eye health condition survey interface according to an embodiment of the present invention.

Referring to FIGS. 13A and 13B, the eye health condition survey interface (hereinafter referred to as a survey interface) according to an embodiment may be an interface that performs a predetermined question-and-answer process for determining eye health conditions of a subj ect.

In an embodiment, the processor 175 may provide a survey interface based on the display portion 150 as shown in FIG. 13A.

Further, the processor 175 may execute a question-and-answer process for understanding eye health conditions of a subject based on the survey interface provided through the display portion 150.

In addition, in an embodiment, the processor 175 may provide the survey interface based on the display unit 220 of the head-mounted display 200 as shown in FIG. 13B in conjunction with the head-mounted display 200.

Further, the processor 175 may execute a question-and-answer process for understanding eye health conditions of a subject based on the survey interface provided through the display unit 220.

In more detail, in an embodiment, the processor 175 may output a predetermined questionnaire based on the survey interface.

In addition, the processor 175 may acquire an input of a user (in other words, a subject) based on the output questionnaire.

For example, the processor 175 may output a multiple-choice questionnaire related to eye health based on the survey interface and provide the same to a subject.

Further, the processor 175 may acquire an input of a subject for selecting a response (for example, a predetermined number) to the provided multiple-choice questionnaire.

In this connection, according to an embodiment, the processor 175 may acquire the user input in a method such as a touch input to the display portion 150 or a gesture input based on the display unit 220.

In this connection, in an embodiment, the processor 175 may divide the provided questionnaire into predetermined sections and implement the same.

In an embodiment, the processor 175 may divide the questionnaire into sections such as gender, age, life pattern, recent subjective symptoms, chronic disease, and/or eye care habits.

In addition, in an embodiment, the processor 175 may implement a questionnaire including at least one or more query items for each section divided as above.

For example, the processor 175 may include: a gender selection query item in the section of gender; an age input query item in the section of age; a query item such as long-term driving, stress, frequent and heavy use of electronic devices, overtime work, and/or outdoor activities in the section of lifestyle pattern; a query item such as dyslexia, congestion, dry eyes, foreign body sensation, irritation, burning sensation, blurred vision, pain, astigmatism, headache, crooked vision, blurred vision, decreased vision, double vision, eye floaters, and/or ophthalmodonesis in the section of recent subjective symptoms; a query item such as diabetes, hypertension and/or eye disease (such as amblyopia, strabismus, glaucoma, retinal abnormalities, optic nerve abnormalities, macular degeneration, color blindness, astigmatism, cataracts, dry eye and/or presbyopia) in the section of chronic disease; and a query item such as intake of good medicine for eyes, intake of good food for eyes, visits to an ophthalmologist in the past year and/or glasses worn in the section of eye care habits of the questionnaire.

As such, the processor 175 performs a preliminary investigation on eye health conditions of a subject based on the survey interface. Accordingly, it is possible to acquire consistent and reliable base data capable of analyzing the eye health condition of the subject through a systematic measurement method, and to derive highly reliable analysis results based thereon.

In addition, in an embodiment, the processor 175 may determine an eye health measurement method based on the survey interface provided as above. (S 103)

In detail, in an embodiment, the processor 175 may determine a type of eye health measurement content (hereinafter referred to as an eye health measurement method) customized for a subject based on an input of the corresponding subject based on the survey interface.

Herein, the eye health measurement content according to an embodiment is content that provides a process for measuring eye health conditions according to predetermined parameters (for example, stereoscopic vision, eyesight, macular degeneration, or extrinsic ocular muscles). In an embodiment, at least one piece of eye health measurement content may be constructed on the eye health measurement service in order to measure the eye health conditions from various viewpoints (parameters).

In addition, in an embodiment, the eye health measurement content may be implemented based on virtual reality.

In an embodiment, the eye health measurement content may be implemented as eyesight measurement, visual field measurement, astigmatism measurement, macular degeneration measurement, color blindness measurement, stereopsis measurement, diplopia measurement, contrast sensitivity measurement, extrinsic ocular muscle measurement, or dynamic eyesight measurement content based on virtual reality.

In an embodiment, the processor 175 may determine an eye health measurement method optimized for a corresponding subject among various types of eye health measurement content, based on an input of a subject based on the aforementioned survey interface.

In detail, in an embodiment, the processor 175 may determine eye health conditions of a subject based on an input of the subject based on the survey interface.

Specifically, the processor 175 may calculate an eye health score for a corresponding subject based on an input of the subject.

In this connection, in an embodiment, the processor 175 may separately calculate the eye health score for a subject for each piece of eye health measurement content.

In an embodiment, the processor 175 may acquire response information according to an input of a subject for each query item of a questionnaire provided through the survey interface.

Further, the processor 175 may give a score for each piece of eye health measurement content based on the acquired response.

For example, in the case of a query item in the section of recent subjective symptoms, when the response information of a subject includes `dyslexia' checked, the processor 175 may give, among eye health measurement methods, a score of '+30' to eyesight measurement content, give a score of '+20' to astigmatism and extrinsic ocular muscle measurement content, and give a score of'+10' to the eyesight measurement content.

In another embodiment, when the response information of a subject in a query item of the section of recent subjective symptoms includes 'congestion' checked, the processor 175 may give, among eye health measurement methods, a score of '+30' to eyesight measurement content, give a score of'+30' to pupil measurement content, and give a score of'+10' to contrast sensitivity measurement content.

In yet another embodiment, when the response information of a subject in a query item of the section of recent subjective symptoms includes `crooked vision' checked, the processor 175 may give, among eye health measurement methods, give a score of '+30' to eyesight measurement content and macular degeneration measurement content.

In addition, in an embodiment, the processor 175 may calculate an eye health score for each piece of eye health measurement content for a corresponding subject based on the score for each piece of eye health measurement content given as above based on the survey response of a subject.

For example, the processor 175 may calculate an eye health score for first eye health measurement content by performing pattern analysis based on the given score configuring the score for the first eye health measurement content.

For example, when there is no given score that is '+10 points or higher' among the given scores configuring the first eye health measurement content score, the processor 175 may set an eye health score for the first eye health measurement content as an eye health score within a preset range of 'good.'

In another embodiment, when there is a score of '+10 points or higher and less than +25 points' and there is no given score of '+25 points or higher among the given scores configuring the first eye health measurement content score, the processor 175 may set an eye health score for the first eye health measurement content as an eye health score within a preset range of 'normal.'

In yet another embodiment, when there is one or more assigned scores of '+25 points or higher' among the assigned scores configuring the first eye health measurement content score, the processor 175 may set an eye health score for the first eye health measurement content as an eye health score within a preset range of 'poor.'

In addition, in an embodiment, the processor 175 may predict a suspected disease for a subject based on an eye health score for each piece of eye health measurement content calculated as above.

For example, when the eye health score for eyesight, visual field and macular degeneration measurement content is within the poor range, the processor 175 may predict amblyopia, glaucoma, cataract, and/or macular degeneration as suspected diseases for a corresponding subject.

In addition, in an embodiment, the processor 175 may determine an eye health measurement method to be performed on a corresponding subject based on the predicted suspected disease.

In detail, in an embodiment, the processor 175 may determine at least one piece of eye health measurement content as an eye health measurement method to be applied to a corresponding subject according to the predicted suspected disease.

For example, when the suspected disease includes amblyopia, the processor 175 may determine the eyesight measurement content as an eye health measurement method to be applied to a subject.

For another example, when the suspected disease includes macular degeneration, the processor 175 may determine the macular degeneration measurement content as an eye health measurement method to be applied to a subject.

In yet another embodiment, when the suspected disease includes glaucoma, the processor 175 may determine visual field measurement, astigmatism measurement and pupil measurement content as an eye health measurement method to be applied to a subject.

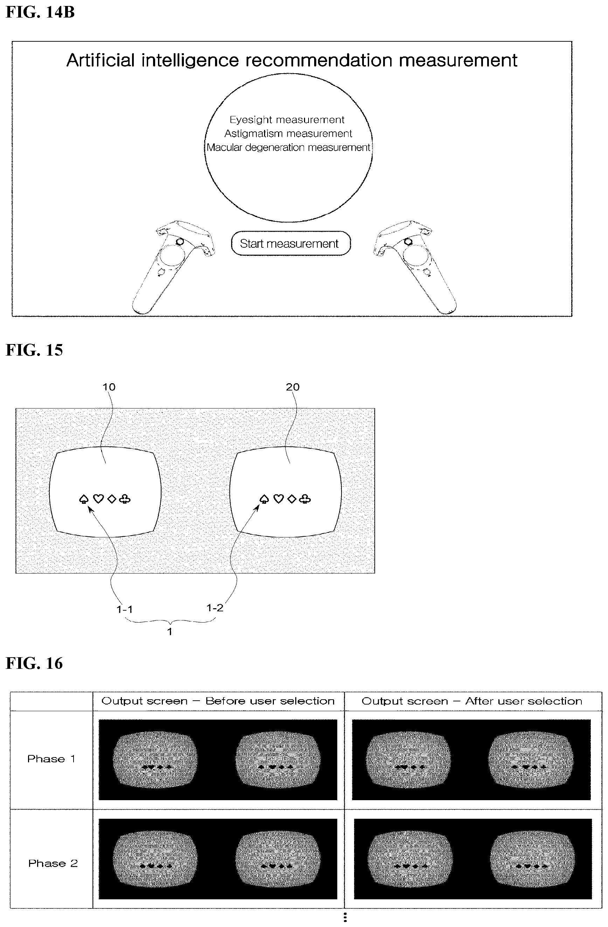

FIGS. 14A and 14B are examples of states displaying eye health condition survey information according to an embodiment of the present invention.

In this connection, referring to FIGS. 14A and 14B, in an embodiment, the processor 175 may generate eye health condition survey information based on the information acquired as above based on an input of a subject to the survey interface.

Further, the processor 175 may display and output the generated eye health condition survey information and provide the same to a user (in an embodiment, a subject and/or measurer).

Herein, the eye health condition survey information according to an embodiment is information acquired by pre-surveying the eye health conditions of a corresponding subject based on an input of a subject to the survey interface. In an embodiment, pattern analysis result information (for example, good, normal, or poor) according to eye health scores for each piece of eye health measurement content related to a corresponding subject, suspected symptom information, and/or determined eye health measurement method information may be included.

In an embodiment, the processor 175 may use the display portion 150 to output eye health condition survey information for a subject, as shown in FIG. 14A.

In addition, in an embodiment, the processor 175 may output eye health condition survey information for a subject based on the display unit 220 in conjunction with the head-mounted display 200 as shown in FIG. 14B.

As such, the processor 175 understands the current eye health condition of a subject based on the results of the survey analysis, and select and provide an eye health measurement method necessary for the corresponding measurement subject based thereon. Accordingly, it is possible to conduct a customized eye health measurement process optimized for each subject, and thus, it is possible to reduce the cost or effort required to measure eye health conditions, as a result, the efficiency of the eye health measurement service can be improved.

In addition, in an embodiment, the processor 175 may execute eye health condition measurement based on the eye health measurement method determined as above. (S 105)

In detail, in an embodiment, the processor 175 may determine, among a plurality of eye health measurement methods, at least one eye health measurement method to be performed on a subject, and measure eye health conditions based on the determined at least one eye health measurement method.

In the following embodiment, for effective description, among a plurality of eye health measurement methods, stereopsis measurement content is set as the first eye health measurement method, and eye health measurement content is set as a second eye health measurement method to execute eye health measurement. However, this is merely an embodiment, and according to an embodiment, at least one eye health condition measurement based on various pieces of eye health measurement content (for example, visual field measurement, astigmatism measurement, macular degeneration measurement, color blindness measurement, diplopia measurement, contrast sensitivity measurement, extrinsic ocular muscle measurement and/or dynamic eyesight measurement) may be performed.

In an embodiment, the processor 175 may measure eye health conditions of a subject based on the determined first eye health measurement method.

In detail, in an embodiment, the processor 175 may set stereopsis measurement content as the first eye health measuring method, and may measure eye health conditions of a subject based thereon.

Herein, the stereopsis measurement content according to an embodiment may be content that measures whether an accurate depth may be recognized using the parallax of both eyes.

More specifically, in an embodiment, the processor 175 may execute eye health measurement based on stereopsis measurement content.

Further, the processor 175 may display at least three optotypes based on an virtual reality image in conjunction with the head-mounted display 200 based on the executed stereopsis measurement content.

For example, the processor 175 may display four optotypes arranged in a line on an virtual reality image.

In addition, in an embodiment, the processor 175 may provide a stereopsis measurement interface (a first eye health condition measurement interface) capable of selecting a displacement optotype, which is an optotype having a different depth from other optotypes, among a plurality of displayed optotypes.

In detail, the processor 175 may display a plurality of optotypes including one displacement optotype on a virtual reality image, and may provide a stereopsis measurement interface for selecting a displacement optotype among the plurality of displayed optotypes.

In this connection, in an embodiment, the processor 175 may adjust the position and/or size of a first optotype to be displayed as a displacement optotype in order to implement the displacement optotype having a different depth from other optotypes on a virtual reality image.

FIGS. 15 and 16 are examples of diagrams for explaining stereopsis measurement content according to an embodiment of the present invention.

Referring to FIGS. 15 and 16, in an embodiment, in general, when a virtual reality image is output in conjunction with the head-mounted display 200, the processor 175 may output the virtual reality image based on a first display region 10 corresponding to the left eye region of a subject wearing the head-mounted display 200 and a second display region 20 corresponding to the right eye region of the subject.

In this connection, in an embodiment, when trying to implement the depth of a first optotype 1 set as a displacement optotype differently from the other optotypes among a plurality of optotypes in the virtual reality image, the processor 175 may set and output different positions between a first optotype 1-1 (hereinafter referred to as a 1-1 optotype) output through the first display region 10 and a first optotype 1-2 (hereinafter referred to as a 1-2 optotype) output through the second display region 20.

In this connection, the processor 175 may output the 1-1 optotype and the 1-2 optotype in a different color to be distinguished from other optotypes on a corresponding display region. For example, the processor 175 may display the 1-1 optotype and the 1-2 optotype in red color and display and output the other optotypes in black color.

Herein, the difference in position between the 1-1 optotype 1-1 displayed on the first display region 10 and the 1-2 optotype 1-2 displayed on the second display region 20 may mean that the display coordinate value for the 1-1 optotype 1-1 in the first display region 10 is different from the display coordinate value for the 1-2 optotype 1-2 in the second display region 20 from each other with respect to the 1-1 optotype 1-1 and the 1-2 optotype 1-2 sharing the same 3-dimensional coordinate value (in other words, a 3-dimensional coordinate value of the first optotype 1).

In detail, in an embodiment, the processor 175 may set the display position of each of the 1-1 optotype 1-1 and the 1-2 optotype 1-2 according to a predetermined distance (in other words, depth) to be implemented between the eyeball of a subject wearing the head-mounted display 200 and the first optotype 1.

According to the distance between the eyeballs of a person (in other words, the left and right eyes) and an object (in an embodiment, an optotype) located at a certain distance apart, the position of the object recognized by the left eye of the person and the position of the object recognized by the right eye of the person are recognized differently (in other words, parallax occurs), so that it may be based on the principle that the person may perceive the depth (in other words, perspective) for the object.

Depending on an embodiment, considering that the positions of the left and right eyes are variable depending on a subject, the processor 175 may replace the left and right eyes of a subject with the first and second lenses that may be included in the optical unit 240 of the head-mounted display 200 in order to standardize the same.

In the following description, the left and right eyes of a subject will be described based on an embodiment in which the first and second lens are replaced, respectively, without being limited thereto.

In detail, the processor 175 according to an embodiment may determine a viewing angle at which a subject recognizes the first optotype 1, in other words, an angle of stereopsis, according to the distance between the first lens and the second lens of the head-mounted display 200 (hereinafter referred to as binocular distance) and the distance between the first and second lenses and the first optotype 1 gazed at by the subject.

More specifically, the processor 175 may determine a first angle of stereopsis for recognizing the first optotype 1 at a first distance from the first lens, which is a different distance from other optotypes, and a second angle of stereopsis for recognizing the first optotype 1 at a first distance from the second lens.

The angle of stereopsis according to the distance is preset and stored in a memory, and the processor 175 may determine the angle of stereopsis according to the distance change for each angle of stereopsis presently stored in the memory.

In addition, in an embodiment, the processor 175 may set the positions of the 1-1 optotype 1-1 and the 1-2 optotype 1-2 based on the determined angle of stereopsis.

Specifically, the processor 175 may derive a first position on the second display region 20 capable of realizing the first angle of stereopsis determined as above.

Further, the processor 175 may set the derived first position to the position of the optotype 1-2 that is the first optotype 1 displayed on the second display region 20.

In the same manner, in an embodiment, the processor 175 may derive a second position at which the second angle of stereopsis determined as above may be implemented on the first display region 10, and the derived second position may be set to the position of the 1-1 optotype 1-1 that is the first optotype 1 displayed on the first display region 10.

In other words, in an embodiment, the processor 175 may adjust the position of the 1-2 optotype 1-2 to implement the first angle of stereopsis corresponding to the left eye on the region recognized by the left eye (the second display region 20), and adjust the position of the 1-1 optotype 1-1 to implement the second angle of stereopsis corresponding to the right eye on the region recognized by the right eye (the first display region 10).

In addition, in an embodiment, the processor 175 may display the first optotype 1 on the first display region 10 and the second display region 20 based on the positions of the 1-1 optotype 1-1 and the 1-2 optotype 1-2 set as above.

Thus, in an embodiment, the processor 175 may display the first optotype 1 displayed by combining the 1-1 optotype 1-1 and the 1-2 optotype 1-2 as a displacement optotype present on a first distance (depth) different from other optotypes based on the parallax effect based on the positional difference between the 1-1 optotype 1-1 and the 1-2 optotype 1-2.

In other words, in an embodiment, in order to implement a displacement optotype (in other words, the first optotype 1) having a depth different from other optotypes on a virtual reality image, the processor 175 may set and output different positions between the 1-1 optotype 1-1 of the virtual reality image output through the first display region 10 and the 1-2 optotype 1-2 of the virtual reality image output through the second display region 20.

In another aspect, the processor 175 may determine 3-dimensional coordinates of optotypes to be displayed in a 3-dimensional virtual space where a virtual reality image is displayed for measurement of stereoscopic vision.

In this connection, the processor 175 may randomly determine one of a plurality of optotypes as a displacement optotype so as to check stereoscopic vision.

For example, the processor 175 may randomly determine one of the first to fourth optotypes as a displacement optotype when displaying the first to fourth optotypes in a row from the left.

In addition, the processor 175 may determine a y-axis coordinate of the displacement optotype differently from a y-axis coordinate of the other optotypes in a 3-dimensional virtual space. Herein, the y-axis means depth and means the direction in which a subject looks at the 3-dimensional virtual space, so that the y-axis coordinates set differently may be understood as meaning that the aforementioned distance is changed.

In other words, the remaining optotypes may be displayed at the same depth in the virtual space by matching the y-axis coordinates, and only the displacement optotype may be displayed at different depths by changing the y-axis coordinates.

In addition, the x-axis coordinates and/or z-axis coordinates of the optotypes are made to be different to be displayed at different positions at the same depth. In an embodiment, the processor 175 may display the optotypes in a row by making only the x-axis coordinates of the optotypes different and matching the z-axis coordinates.

After determining all the 3-dimensional coordinates of the optotypes in the virtual space for the measurement of stereoscopic vision, the processor 175 may determine the display coordinates of each optotype in the first display region 10 and the second display region 20 so that the optotypes are displayed according to the 3-dimensional coordinates.

In this connection, since the first display region 10 and the second display region 20 are two-dimensional planes, only the x-axis and z-axis coordinates may be changed. Accordingly, in order to display optotypes at different depths, the parallax between the left eye and the right eye needs to be used.

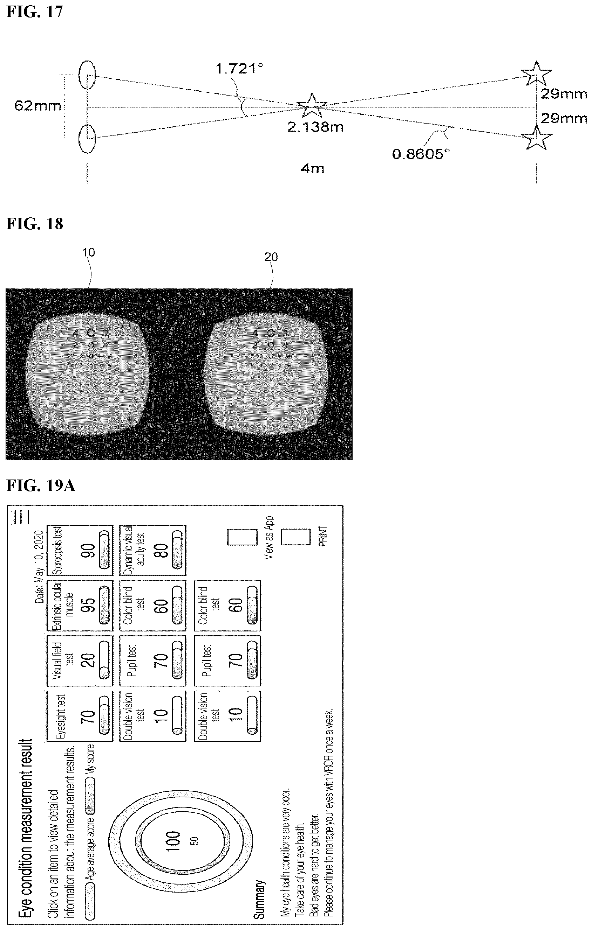

FIG. 17 is an example of a diagram for explaining a method of implementing a displacement optotype according to an embodiment of the present invention.

In detail, referring to FIG. 17, the processor 175 may calculate angle of stereopsis of the left and right eyes to be displayed on the 3-dimensional coordinates when x1, y1, and z1 are the 3-dimensional coordinates of the displacement optotype.

Herein, as can be understood with reference to FIG. 17, the angle of stereopsis may be determined based on the distance between the first lens corresponding to the left eye and the second lens corresponding to the right eye, the distance between a display region and lens, and the 3-dimensional coordinates of the displacement optotype.

Further, the processor 175 may determine 2-dimensional display coordinates (for example, x-axis coordinates and z-axis coordinates) of the first display region 10 for displaying a displacement optotype on 3-dimensional coordinates based on the calculated angle of stereopsis, and 2-dimensional display coordinates (for example, x-axis coordinates and z-axis coordinates) of the second display region 20.

In other words, by making the x-axis and z-axis coordinates in the first display region 10 and the x-axis and z-axis coordinates in the second display region 20 different from each other, a parallax between the left eye and the right eye occurs. Accordingly, the depth (y-axis coordinate) of the displacement optotype may be determined.

The processor 175 may obtain the x-axis and z-axis coordinates of the first display region 10 and the second display region 20 so that the remaining optotypes may be displayed on the 3-dimensional coordinates in the same manner. In this connection, the depths of the remaining optotypes may all be the same.

Thus, the processor 175 may generate a predetermined parallax when a subject gazes at the 1-2 optotype displayed at the first position and the 1-1 optotype displayed at the second position from each of the first lens and the second lens. Accordingly, a predetermined depth (distance) effect corresponding to the generated parallax may be implemented.

In addition, in an embodiment, the processor 175 may adjust the position values set for each of the 1-1 optotype 1-1 and the 1-2 optotype 1-2 according to the gradual depth (distance) to be implemented.

For example, further referring to FIG. 17, in order to implement a parallax for implementing a displacement optotype (the first optotype 1) that is as close as 3000 arcseconds at a distance of 4 m, the processor 175 may move the 1-1 optotype 1-1 of the first display region 10 to the right by 29 mm from the reference point of the other optotypes, and moves the 1-2 optotype 1-2 of the second display region 20 to the left by 29 mm.

In this connection, in an embodiment, the processor 175 may also adjust the size of the first optotype 1 as the depth of the first optotype 1 to be displayed as a displacement optotype changes.

In an embodiment, the processor 175 may change the size of the first optotype 1 in inverse proportion to the depth (depth) of the first optotype 1.

Returning again, in an embodiment, the processor 175 may output a plurality of optotypes including the displacement optotype implemented as above as a virtual reality image, and may provide a stereopsis measurement interface for selecting the displacement optotype from among a plurality of output optotypes.

Further, the processor 175 may perform stereopsis measurement step by step while sequentially reducing the depth difference between the displacement optotype and the remaining optotypes according to a selection input of a subject.

In detail, when a subject selects and matches the first displacement optotype, the processor 175 may determine a second depth difference smaller than the first depth difference between the first displacement optotype and the other optotypes and one of the optotypes as a second displacement optotype. As described above, the second displacement optotype may be randomly determined.

Further, the processor 175 may determine the 3-dimensional virtual coordinates of the second displacement optotype and the 3-dimensional virtual coordinates of the other optotypes to have a second depth difference from the other optotypes.

In addition, in order to display the determined 3-dimensional virtual coordinates, the processor 175 may calculate an angle of stereopsis for each optotype based on the 3-dimensional virtual coordinates of the optotypes, the distance between the first lens and the second lens, and the distance between the display region and the lens.

Next, the processor 175 may calculate 2-dimensional display coordinates for displaying optotypes in the first display region 10 and the second display region 20 according to the calculated angle of stereopsis.

Further, the processor 175 may display optotypes on the 2-dimensional display coordinates calculated on each of the first display region 10 and the second display region 20, and display the optotypes on predetermined 3-dimensional virtual coordinates.

Next, the processor 175 may select a displacement optotype according to an input of a subject. When the displacement optotype is matched, the depth difference is further reduced, and then the measurement of stereopsis is repeated to precisely detect the depth that may be recognized by a subject, that is, stereoscopic vision.

In addition, the processor 175 may acquire stereopsis measurement result scores based on an input of a subject to the provided stereopsis measurement interface.

In an embodiment, the processor 175 may calculate a correct answer rate for accurately selecting a displacement optotype based on an input of a subject to the stereopsis measurement interface, and acquire a stereopsis measurement result score in proportion to the calculated correct answer rate.

As such, the processor 175 changes the depth of the optotype by adjusting the position and/or size of the optotype displayed as a virtual reality image, and performs a stereopsis measurement process based thereon. Accordingly, the principles of stereopsis measurement based on analog devices such as polarized glasses can be digitized and easily implemented.

In an embodiment, the processor 175 may measure eye health conditions of a subject based on the second eye health measurement method determined based on the survey interface.

In detail, in an embodiment, the processor 175 may set the eyesight measurement content as the second eye health measurement method, and may measure the eye health conditions of the subject based thereon.

Herein, the eyesight measurement content according to an embodiment may be content for measuring eyesight and focus ability.

More specifically, in an embodiment, the processor 175 may execute an eye health measurement based on the eyesight measurement content.

FIG. 18 is an example of a diagram for explaining eyesight measurement content according to an embodiment of the present invention.

In addition, referring to FIG. 18, in an embodiment, the processor 175 may display an eyesight measurement table based on a virtual reality image in conjunction with the head-mounted display 200 based on the executed eyesight measurement content.

Herein, the eyesight measurement table according to an embodiment is a table used to measure eyesight, and may be a table printed with various characters or pictures (codes) enlarged or reduced according to a prescribed criterion.

In detail, in an embodiment, the processor 175 may display an eyesight measurement table located on a predetermined depth based on a virtual reality image.

In addition, in an embodiment, the processor 175 may provide, based on the displayed eyesight measurement table, an eyesight measurement interface (a second eye health condition measurement interface) through which a subject may select a target symbol, which is a predetermined symbol required to be selected from among a plurality of symbols in the corresponding eyesight measurement table.

In more detail, in an embodiment, the processor 175 may determine a target symbol automatically randomly selected by a measurer or the processor 175 from among at least one symbol included in the displayed eyesight measurement table.

In addition, the processor 175 may inform a subject of the determined target symbol.

In an embodiment, the measurer who measures eye health conditions may determine, as a target symbol, one randomly selected among the symbols in the eyesight measurement table displayed on a virtual reality image.

In this connection, the processor 175 may acquire information on a target symbol randomly determined by the aforementioned measurer.

In an embodiment, the processor 175 may acquire information about a target symbol selected by a measurer based on the input portion 160.

In addition, in an embodiment, the processor 175 may output audio data for a corresponding target symbol and provide the same to a target based on the target symbol information acquired as described above.

For example, when '3' is determined as the target symbol, the processor 175 may output audio data for the corresponding target symbol providing audio data of a Korean transliteration of number '3' and provide the same to a subject.

In another embodiment, the processor 175 may automatically and randomly select a target symbol among the symbols in the eyesight measurement table.

In addition, the processor 175 may output audio data for the target symbol automatically selected as described above and provide the same to a subject.

For example, when the target symbol is determined to be '3' by its own process, the processor 175 may output audio data for the corresponding target symbol that provides voice data of a Korean transliteration of number '3' and provide the same to a subject.

Alternatively, in another embodiment, the processor 175 displays a graphic image of the automatically selected target symbol on a depth perceptible by a corresponding subject (for example, based on the uncorrected eyesight value according to the previous eyesight measurement of the corresponding subject). Accordingly, the corresponding target symbol may be informed to the subject.

For example, when the target symbol is determined to be '3' by its own process, the processor 175 may display a graphic image (herein, a graphic image representing the number 3) representing the corresponding target symbol on a predetermined depth (in an embodiment, a depth perceptible by a corresponding subject) in a virtual reality image and provide the same to the corresponding subject.

Subsequently, in an embodiment, the processor 175 may acquire an eyesight measurement result score based on an input of a subject to the eyesight measurement interface provided as described above.

In detail, in an embodiment, the processor 175 may acquire a response of a subject to the target symbol determined as above based on the eyesight measurement interface.

In an embodiment, the processor 175 may acquire an input of a subject to select a symbol having a shape corresponding to a target symbol provided with audio data from among at least one symbol (for example, a character or a code) in an eyesight measurement table displayed on a predetermined depth of a virtual reality image based on the eyesight measurement interface.

Further, the processor 175 may acquire a response to a corresponding target symbol based on the acquired input of the subject.

In another embodiment, the processor 175 may acquire an input of a subject to select a symbol having a shape corresponding to a target symbol displayed on a predetermined first depth (in an embodiment, a depth clearly perceptible by a corresponding subject) in a virtual reality image from among at least one symbol in an eyesight measurement table displayed on a predetermined second depth (in an embodiment, a predetermined depth set for testing eyesight measurement) of the virtual reality image based on the eyesight measurement interface.

Further, the processor 175 may acquire a response to a corresponding target symbol based on the input of the subject acquired as above.

In addition, in an embodiment, the processor 175 may repeatedly perform a series of processes of randomly determining a target symbol and acquiring a response of a subject to the determined target symbol as described above.

In this connection, in an embodiment, the processor 175 may cause the eyesight measurement table output as a virtual reality image to be displayed while gradually changing a depth as eyesight measurement progresses based on user (in an embodiment, a measurer) settings and/or automated processes.

In an embodiment, the processor 175 may acquire a response to a target symbol based on the eyesight measurement table displayed on the first depth in a first measurement phase when the eyesight measurement content is executed.

Thereafter, in the first measurement phase, when a subject correctly selects the symbol corresponding to a target symbol, the processor 175 may output an eyesight measurement table on a second depth located farther than the first depth in a second measurement phase.

Further, the processor 175 may perform a process of acquiring a response to a target symbol based on the eyesight measurement table displayed on the second depth.

In addition, the processor 175 may repeatedly perform the above process up to a predetermined n^{th} phase (n=1, 2, 3, ...).

In an embodiment, when a subject does not select a symbol corresponding to a target symbol in the first measurement phase, the processor 175 may output an eyesight measurement table on the first depth also in the second measurement phase.

Further, the processor 175 may acquire a response to a second symbol in a second phase, which is different from the target symbol in a first phase, based on the eyesight measurement table displayed again on the first depth as described above.

In addition, the processor 175 may repeatedly perform the above process up to a predetermined n^{th} phase (n=1, 2, 3, ...).

As such, the processor 175 outputs an eyesight measurement table as a virtual reality image, and proceeds with gradual eyesight measurement by adjusting the depth of the outputted eyesight measurement table, thereby overcoming the constraints that need to be endured when using a physical eyesight measurement table (for example, availability of eyesight measurement table equipment, variability of a distance between a subject and an eyesight measurement table, limited number, size and/or position of symbols on the eyesight measurement table) and measuring eyesight of a subject more conveniently.

In addition, in an embodiment, the processor 175 may calculate an eyesight measurement result score based on the responses acquired by performing the above multiple times.

In an embodiment, the processor 175 may calculate an eyesight measurement result score of a corresponding subject in proportion to a correct answer rate at which a target symbol is accurately selected, based on an input of the subject based on the eyesight measurement interface.

However, in general, in a method of displaying an eyesight measurement table through an image and providing the same to a subject, when the resolution of an instrument that displays the corresponding eyesight measurement table (in an embodiment, the head-mounted display 200) is low, a situation may occur in which an eyesight measurement result is determined under the influence of factors unrelated to the eyesight of a subject because the eyesight measurement table is not clearly displayed.

Thus, in an embodiment of the present invention, the processor 175 may set the minimum size of the displayed eyesight measurement table according to the resolution of the head-mounted display 200 outputting a virtual reality image.

In an embodiment, the processor 175 may set the minimum size of the output eyesight measurement table in inverse proportion to the resolution of the linked head-mounted display 200.

In other words, in an embodiment, the processor 175 may set the minimum size of the eyesight measurement table output based on the head-mounted display 200 to be smaller as the resolution of the head-mounted display 200 is higher, and vice versa.

In addition, in an embodiment, the processor 175 may adjust a depth of the eyesight measurement table in the corresponding virtual reality image within a range that does not exceed a set minimum size.

For example, when an eyesight measurement table is displayed at a second depth having a greater depth than the first depth (in other words, located at a greater distance) after displaying the eyesight measurement table having a predetermined size at the first depth, the processor 175 may determine the depth of the second depth within a range in which the eyesight measurement table does not become smaller than a minimum size set for the corresponding eyesight measurement table.

In addition, in an embodiment, in the case of the eyesight measurement table displayed in the minimum size, the processor 175 may convert the form of symbols (for example, text and/or picture (code)) in the corresponding eyesight measurement table into a picture (code) form and provide the same.

For example, the processor 175 may change the character symbols in the eyesight measurement table displayed in a minimum size into predetermined picture (code) symbols and provide the same.

In this way, the processor 175 displays the symbols in the eyesight measurement table only in the form of pictures (codes) that are less affected by resolution than text, thereby minimizing the influence of the resolution of the head-mounted display 200 that outputs a virtual reality image in a situation where the eyesight measurement table of the minimum size is displayed.

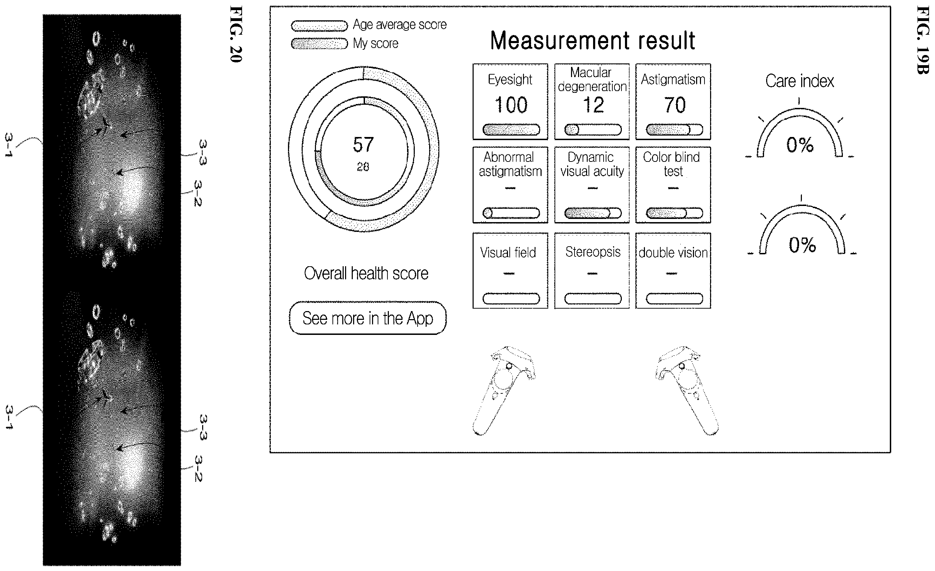

FIGS. 19A and 19B are examples of states displaying eye health condition measurement results according to an embodiment of the present invention.

In addition, referring to FIGS. 19A and 19B, in an embodiment, the processor 175 may display a result of eye health condition measurement (in an embodiment, stereopsis measurement and/or eyesight measurement) executed as described above. (S107)

In other words, in an embodiment, the processor 175 may provide eye health condition information for a subject as a result, based on at least one piece of eye health condition measurement executed.

Herein, the eye health condition information according to an embodiment is information providing an analysis result of eye health conditions of a subject estimated based on an eye health measurement service. In an embodiment, the eye health condition information may include result scores for each piece of eye health condition measurement performed on a subject (in an embodiment, stereopsis measurement result scores and/or eyesight measurement result scores), an integrated score calculated by totaling the result scores and the eye health score acquired based on the survey interface (for example, the result score corrected by reflecting the eye health score for the subject's age and/or eye care habits), and/or predictive suspected disease information derived based on a user input to the survey interface.

In detail, in an embodiment, the processor 175 may acquire eye health condition information for a subject based on an input of the subject to the eye health condition measurement interface (in an embodiment, a stereopsis measurement interface and/or an eyesight measurement interface) and the survey interface provided in an eye health condition measurement process.

In this connection, the processor 175 may provide more precisely analyzed result information based on a plurality of pieces of eye health condition information when there is a plurality of pieces of eye health condition measurement data depending on a subject.

For example, the processor 175 performs an analysis based on a plurality of pieces of eye health condition information acquired by measuring eye health condition multiple times, thereby more accurately predicting a disease such as glaucoma (in other words, a disease that needs to be identified over a long period of time), which is difficult to accurately understand only with a one-time measurement of eye health conditions.

Thus, the processor 175 may further improve the performance of the eye health measurement service.

In addition, in an embodiment, the processor 175 may output the acquired eye health condition information based on the display portion 150 as shown in FIG. 19A.

In addition, the processor 175 may output eye health condition information of a subject through the display unit 220 in conjunction with the head-mounted display 200 as shown in FIG. 19B.

In addition, in an embodiment, the processor 175 may store and manage the acquired eye health condition information by making the same into a database for each subject.

Thus, the processor 175 may digitize result data of eye health condition measurement and conveniently manage the same.

In addition, in an embodiment, the processor 175 may provide prescription content according to the eye health condition measurement result. (S109)

Herein, the prescription content according to an embodiment may be content data provided for the purpose of improving eye health conditions of a subject based on the eye health condition measurement result (in other words, eye health condition information).

In an embodiment, the prescription content may include eye healing images and/or information on recommended nutritional supplements.

In detail, in an embodiment, the processor 175 may provide an eye healing image according to the eye health condition information of a subject.

In this connection, the provided eye healing image may be implemented as an ultrahigh-definition image (for example, 4K image) in order to minimize eye fatigue and maximize healing effects.

In an embodiment, the processor 175 may provide, based on the eye health condition information of a subject, a first eye healing image capable of assisting a movement of the intrinsic ocular muscles and/or the extrinsic ocular muscles when it is determined that the subject needs movement of the intrinsic ocular muscles and/or the extrinsic ocular muscles.

In other words, in an embodiment, the first eye healing image may be an image capable of assisting a movement of intrinsic ocular muscles and/or the extrinsic ocular muscles based on virtual reality.

FIGS. 20 to 23 are examples of diagrams for explaining an eye healing image according to an embodiment of the present invention.

In detail, referring to FIGS. 20 to 23, in an embodiment, the processor 175 may display objects implemented with at least two different depths based on the first eye healing image.

In this connection, in an embodiment, the processor 175 may change and display the depth and position of at least two or more displayed objects according to a predetermined criterion (for example, a preset pattern). In other words, in an embodiment, the processor 175 may induce the movement of the intrinsic ocular muscles and/or the extrinsic ocular muscles of a subject by allowing an object to move freely in a 3-dimensional space and making eyeball movement according to the subject's looking at the moving object.

In addition, in an embodiment, the processor 175 may apply or cancel blur processing for each object according to a predetermined criterion (for example, a preset period).

For example, the processor 175 may periodically apply blur processing to make each object blurry or cancel the blur processing to make each object clearer.

In this connection, considering that it is generally desirable to track and gaze at a predetermined object whose depth changes for the movement of the intrinsic ocular muscles and/or the extrinsic ocular muscles, in an embodiment of the present invention, the processor 175 may cancel blur processing on one of the at least two or more objects displayed in the first eye healing image according to a predetermined pattern and set a first object to be displayed clearly, output such that the depth and position of the de-blurred first object change, and induce a subject who watches the first eye healing image to gaze while tracking the first object.

In more detail, in an embodiment, the processor 175 may cancel blur processing on a randomly determined first object among a plurality of objects in the first eye healing image to display the same clearly.

In addition, the processor 175 may cause the first object, which is clearly displayed, to be displayed while changing the depth according to a predetermined pattern.

For example, the processor 175 may output the first object, which is clearly displayed, as a first object 3-1 whose depth is displayed as a new view - a first object 3-2 whose depth is displayed as a middle view - a first object 3-3 whose depth is displayed as a far view, while being changed according to a predetermined pattern.

In this connection, in an embodiment, the processor 175 may change and output the displayed position of the first object according to a predetermined setting (for example, a preset position pattern) together with the depth change of the first object.

In addition, in an embodiment, the processor 175 may convert the aforementioned first object into any one of the remaining objects except the first object according to a predetermined criterion.

In an embodiment, the processor 175 may select and operate any one of a plurality of objects in the first eye healing image that displays objects implemented with at least two different depths as the first object, and then periodically re-select and operate any one of the objects other than the first object as the first object every predetermined time slice (for example, 1 minute).

For example, the processor 175 may select a first object as the first object among the first object, second object, and third object in the first eye healing image to operate the aforementioned first eye healing image process.

Further, when a predetermined time slice elapses, the processor 175 may select any one of the second object and the third object as the first object and operate the first eye healing image process.

Herein, the processor 175 according to an embodiment may blur the remaining objects except the first object by applying blur processing.

In addition, the processor 175 may prevent a change in the depth or position of the remaining objects from occurring.

In other words, in an embodiment, the processor 175 cancels the blur processing according to a predetermined criterion and outputs the clearly displayed first object while varying the depth and position, and applies the blur processing to the remaining objects except the first object. By blurring the display and not changing the depth and position at the same time, concentration on the first object can be further enhanced, and thus, the movement of the intrinsic ocular muscles and/or the extrinsic ocular muscles on a subject can be improved.

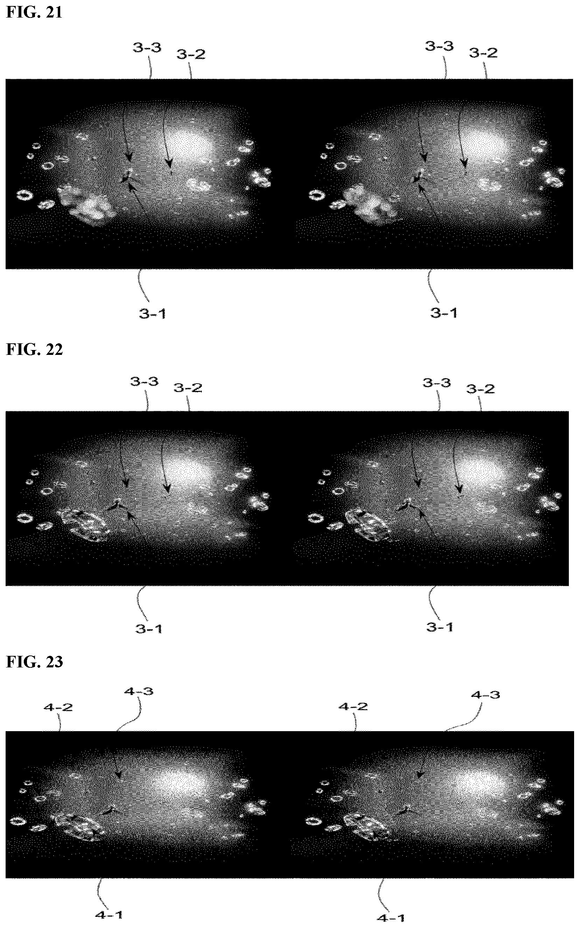

In addition, referring further to FIG. 23, in an embodiment, the processor 175 may further display at least one background objects in addition to a plurality of objects in the eye healing image.

Herein, the background object according to an embodiment may refer to the remaining objects displayed through the eye healing image in addition to a plurality of objects that perform the aforementioned functional operations in the eye healing image.

In detail, in an embodiment, the processor 175 may display at least one background object on an arbitrary position and depth in the eye healing image.

For example, the processor 175 may output a first background object 4-1 displayed in a new view, a second background object 4-2 displayed in a middle view, and a third background object 4-3 displayed in a far view together with at least two or more objects in the eye healing image based on virtual reality.

In this connection, in an embodiment, the processor 175 may equally apply or cancel blur processing according to whether the blur processing is applied to the object corresponding to the background object corresponding to the displayed background object.

In detail, in an embodiment, the processor 175 may detect at least one background object having the same depth as an arbitrary object in the eye healing image.

In addition, the processor 175 may equally apply the blur processing applied to the arbitrary object to the detected background object.

In an embodiment, when blur processing is applied to an arbitrary object, the processor 175 may equally apply the blur processing also to at least one background object having the same depth as the corresponding object.

Also in the opposite case, when blur processing is released for an arbitrary object, the processor 175 may also release the blur processing for at least one background object having the same depth as the corresponding object.

In other words, in an embodiment, the processor 175 may cause a background object around the first object to be clearly displayed among the plurality of objects in the eye healing image to be displayed as clearly as the first object, and may cause a background object around the remaining blurry objects to be displayed blurry like the remaining objects.

Thus, when a subject performs eyeball movement through eye healing images, the processor 175 may more smoothly track the first object that is displayed clearly, and assist in recognizing the depth implemented in the virtual reality space provided by the corresponding eye healing images more easily.

In addition, in this embodiment, the processor 175 makes it easy and convenient to perform the movement of the intrinsic ocular muscles and/or the extrinsic ocular muscles of a subject based on a virtual reality image (the first eye healing image), so that eye care, which is difficult to perform only with eyeball activities in daily life, can be effectively implemented.

In addition, in an embodiment, the processor 175 may provide information on recommended nutritional supplements according to the eye health condition information of a subject.

In detail, in an embodiment, the processor 175 may provide customized information on recommended nutritional supplements that are determined to be necessary for the subject to take according to the eye health condition information of the subject.

In an embodiment, the processor 175 may acquire nutrient information determined to be necessary for the subject based on the eye health condition information of the subject.

For example, the processor 175 may determine that the subject needs lutein and/or zeaxanthin nutrients based on the eye health condition information of the subject.

In addition, in an embodiment, the processor 175 may acquire information on nutritional supplements including nutrients determined to be necessary for the subject in conjunction with an external server (for example, a web server).

For example, the processor 175 may acquire information on nutritional supplements including lutein and/or zeaxanthin nutrients determined to be necessary for the subject in conjunction with the web server.

In addition, in an embodiment, the processor 175 may acquire information on a seller selling a corresponding nutritional supplement (for example, sales site information) in conjunction with an external server (for example, a web server).

In addition, in an embodiment, the processor 175 may generate and provide information on recommended nutritional supplements optimized for a subject based on the acquired information on nutrients, nutritional supplements, and/or seller.

In other words, in an embodiment, the processor 175 provides information on recommended nutritional supplements customized to a subject based on the eye health condition information of the subject, thereby effectively assisting the continuous eye health management in a reasonable manner based on the eye health condition measurement result.

As described above, the eye health measurement system according to an embodiment of the present invention determines an eye health measurement method customized for a measurement subject (in other words, a subject to be measured) based on a survey process related to eye health, and performs an eye health measurement service for the measurement subject based on the determined eye health measurement method. Accordingly, it is possible to acquire consistent and reliable base data capable of analyzing the eye health condition of the subject through a systematic measurement method, and to derive highly reliable analysis results based thereon.

In addition, the eye health measurement system according to an embodiment of the present invention understands the current eye health condition of a measurement subject based on the results of the eye health condition survey analysis, and selects and provides an eye health measurement method necessary for the corresponding measurement subject based thereon. Accordingly, it is possible to conduct a customized eye health measurement process optimized for each subject, and thus, it is possible to reduce the cost or effort required to measure eye health conditions, as a result, the efficiency of the eye health measurement service can be improved.

In addition, the eye health measurement system according to an embodiment of the present invention implements and provides the eye health condition measurement result as a graphic image, so that the eye health condition measurement result can be recognized more intuitively.

### - 3) Eye Health Measurement Computing Device 600 (Computing Device)

In an embodiment of the present invention, an eye health measurement computing device 600 (hereinafter referred to as a computing device) may execute an eye management application capable of implementing an eye health solution service that assists customized eye health management optimized for a measurement subject from a diversified perspective based on the eye health condition measurement result.

In an embodiment, the computing device 600 may include various types (for example, mobile type or desktop type) of computing devices 600 on which an eye management application is installed.

### 1. Mobile Type Computing Device 400

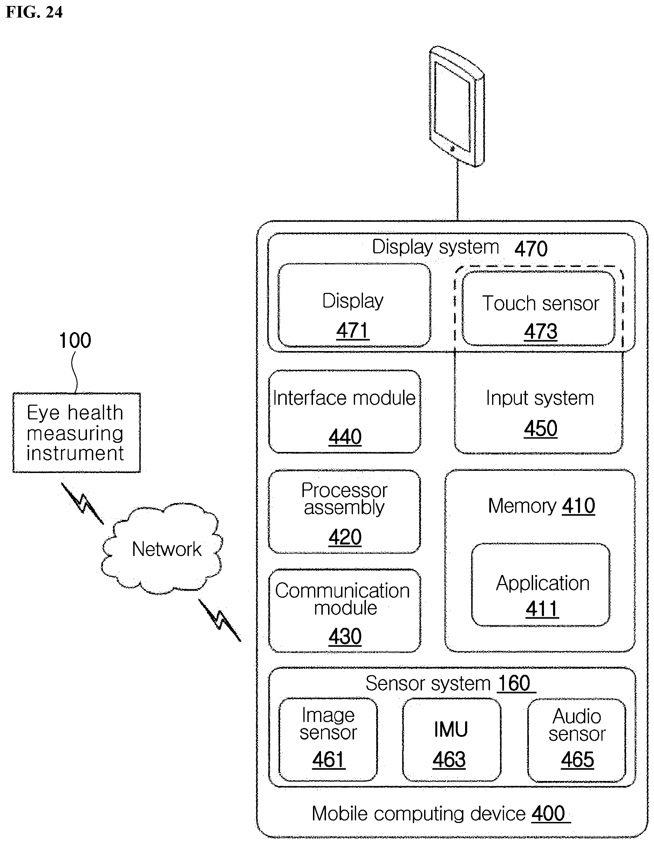

FIG. 24 is an internal block diagram of a mobile type computing device according to an embodiment of the present invention.

Referring to FIG. 24, in an embodiment of the present invention, a mobile type computing device 400 may be a mobile device such as a smart phone or a tablet PC in which an eye management application 411 is installed.

For example, the mobile type computing device 400 may include a smart phone, a mobile phone, a digital broadcast terminal, a PDA (personal digital assistants), a PMP (portable multimedia player), a tablet PC, and the like.

Referring further to FIG. 7, the mobile type computing device 400 according to an exemplary embodiment may include a memory 410, a processor assembly 420, a communication module 430, an interface module 440, an input system 450, a sensor system 460, and a display system 470. These components may be configured to be included within the housing of the mobile type computing device 400.

In detail, the eye management application 411 may be stored in the memory 410, and any one or more of various application programs, data, and instructions for providing an environment in which an eye health measurement service may be implemented may be stored in the eye management application 411.

For example, the memory 410 may include eye health measurement method information (in an embodiment, stereopsis measurement content information and/or visual field measurement content information) and/or eye health condition information.

In other words, the memory 410 may store instructions and data that may be used to generate an eye health measurement service environment.

In addition, the memory 410 may include at least one non-transitory computer-readable storage media and transitory computer-readable storage media. For example, the memory 410 may be various storage devices such as a ROM, an EPROM, a flash driver, and a hard drive, and may be a web storage that performs the storage function of the memory 410 on the Internet.

The processor assembly 420 may include at least one processor capable of executing instructions of the eye management application 411 stored in the memory 410 in order to perform various tasks for implementing an eye health measurement service environment.

In an embodiment, the processor assembly 420 may control overall operations of components through the eye management application 411 of the memory 410 to provide an eye health measurement service.

The processor assembly 420 may include a central processing unit (CPU) and/or a graphic processor unit (GPU). The processor assembly 175 may be implemented by including at least one of application specific integrated circuits (ASICs), digital signal processors (DSPs), digital signal processing devices (DSPDs), programmable logic devices (PLDs), field programmable gate arrays (FPGAs), controllers, micro-controllers, microprocessors, and electronic units for executing other functions.

The communication module 430 may include one or more devices for communicating with other computing devices (for example, the eye health measuring instrument 100 and/or the eye health platform management server 300). This communication module 430 may communicate through a wireless network.

In detail, the communication module 430 may communicate with a computing device storing a content source for implementing an eye health measurement service environment and may communicate with various user input components such as a controller that receives a user input.

In an embodiment, the communication module 430 may transmit/receive various pieces of data related to the eye health measurement service to and from the eye health measuring instrument 100, the eye health platform management server 300, and/or other computing devices 600.

The communication module 430 may wirelessly transmit/receive data with at least one of a base station, an external terminal, and an arbitrary server on a mobile communication network established through a communication device capable of performing technical standards or communication methods for mobile communications (for example, LTE (Long Term Evolution), LTE-A (Long Term Evolution-Advanced), 5G NR (New Radio), and WiFi), or a short-distance communication method.

The sensor system 460 may include various sensors such as an image sensor 461, a position sensor (IMU) 463, an audio sensor 465, a distance sensor, a proximity sensor, and a contact sensor.

The image sensor 461 may capture images and/or video of the physical space around the mobile type computing device 400.

In an embodiment, the image sensor 461 may capture and acquire an image (for example, an eyeball image) related to the eye health measurement service.

In addition, the image sensor 461 may be disposed on the front or/or rear surface of the mobile type computing device 400 to acquire an image by capturing the direction side disposed, and may capture a physical space through a camera disposed toward the outside of the mobile type computing device 400.

The image sensor 461 may include an image sensor device and an image processing module. In detail, the image sensor 461 may process still images or moving images obtained by an image sensor device (for example, CMOS or CCD).

In addition, the image sensor 461 may extract necessary information by processing a still image or moving image acquired through an image sensor device using the image processing module, and transmit the extracted information to a processor.

The image sensor 461 may be a camera assembly including at least one camera. The camera assembly may include a general camera that captures a visible light band, and may further include a special camera such as an infrared camera and a stereo camera.

The IMU 463 may sense at least one of motion and acceleration of the mobile type computing device 400. For example, the IMU 463 may be made of a combination of various position sensors such as an accelerometer, a gyroscope, and a magnetometer. In addition, spatial information on a physical space around the mobile type computing device 400 may be recognized by being linked with the location communication module 430 such as a GPS of the communication module 430.

In addition, the IMU 463 may detect information for detecting and tracking the gaze direction and head motion of a user based on the detected position and direction.

In addition, in some embodiments, the eye management application 411 may use the IMU 463 and the image sensor 461 to determine the position and direction of a user in a physical space or to recognize a feature or object in the physical space.

The audio sensor 465 may recognize sounds around the mobile type computing device 400.

In detail, the audio sensor 465 may include a microphone capable of sensing a voice input of a user of the mobile type computing device 400.

In an embodiment, the audio sensor 465 may receive voice data necessary for an eye health measurement service from a user.

The interface module 440 may communicatively connect the mobile type computing device 400 with one or more other devices. Specifically, the interface module 440 may include wired and/or wireless communication devices compatible with one or more different communication protocols.

Through this interface module 440, the mobile type computing device 400 may be connected to various input/output devices.

For example, the interface module 440 may output audio by being connected to an audio output device such as a headset port or a speaker.

Although the audio output device has been described as being connected through the interface module 440 as an example, an embodiment installed inside the mobile type computing device 400 may also be included.

The interface module 440 may be configured by including at least one of wired or wireless headset ports, external power supply ports, wired or wireless data ports, memory card ports, ports for connecting a device having an identification module, audio input/output (I/O) ports, video I/O ports, earphone ports, power amplifiers, RF circuits, transceivers, and other communication circuits.

The input system 450 may sense an input of a user related to the eye health measurement service (for example, a gesture, voice command, button operation, or other types of input).

In detail, the input system 450 may include a button, a touch sensor, and an image sensor 461 that receives a user motion input.

In addition, the input system 450 may be connected to an external controller through the interface module 440 to receive an input of a user.

The display system 470 may output various pieces of information related to the eye health measurement service as a graphic image.

Such displays may include at least one of a liquid crystal display (LCD), a thin film transistor liquid crystal display (TFT LCD), an organic light emitting diode (OLED), a flexible display, a 3-dimensional display, and an e-ink display.

The components may be disposed within the housing of the mobile type computing device 400, and the user interface may include a touch sensor 473 on a display 471 configured to receive a user touch input.

In detail, the display system 470 may include the display 471 that outputs an image and the touch sensor 473 that senses a touch input of a user.

For example, the display 471 may be implemented as a touch screen by forming a mutual layer structure or being formed integrally with the touch sensor 473. The touch screen may function as a user input portion providing an input interface between the mobile type computing device 400 and a user, and may simultaneously provide an output interface between the mobile type computing device 400 and a user.

### 2. Desktop Type Computing Device 500

Redundant descriptions of the components of a desktop type computing device 500 will be replaced with descriptions of the components of the mobile type computing device 400. Hereinafter, differences from the mobile type computing device 400 will be mainly described.

In another example, the desktop type computing device 500 may further include a device installed with a program for executing an eye health measurement service based on wired/wireless communication, such as fixed desktop PCs, laptop computers, and personal computers such as ultrabooks on which an eye management application is installed.

In addition, the desktop type computing device 500 may include a user interface system and receive a user input (for example, a touch input, a mouse input, a keyboard input, a gesture input, and a motion input using a guide tool).

As an example, the desktop-type computing device 500 may connect the user interface system to at least one device such as a mouse, keyboard, gesture input controller, image sensor (for example, camera), and audio sensor through various communication protocols to acquire a user input.

In addition, the desktop type computing device 500 may be connected to an external output device through a user interface system, and may be connected to, for example, a display device and an audio output device.

In addition, the desktop type computing device 500 according to an exemplary embodiment may include a memory, a processor assembly, a communication module, a user interface system, and an input system. These components may be configured to be included within the housing of the desktop type computing device 500.

The description of the components of the desktop type computing device 500 will be replaced with the description of the components of the mobile type computing device 400.

However, in an embodiment of the present invention, the components shown in FIG. 7 are not essential to implement the computing device 600, so the computing device 600 described herein may have more or fewer components than those listed above.

In addition, according to an embodiment of the present invention, various embodiments may be possible, such as that some of the aforementioned various functional operations performed by the computing device 600 may be performed by the eye health measuring instrument 100 and/or the eye health platform management server 300.

In addition, in the following embodiments, the computing device 600 is described based on the mobile type computing device 400 for effective description, but is not limited thereto.

### - Method for Providing Virtual Reality-Based Eye Health Measurement Service

Hereinafter, a method for providing an eye health measurement service using virtual reality by the eye health measurement system according to an embodiment of the present invention will be described in detail with reference to the accompanying drawings.

In this connection, the contents overlapping with the aforementioned description may be summarized or omitted.

The virtual reality-based eye health measurement system according to an embodiment of the present invention may be implemented based on at least some of the aforementioned eye health measuring instrument 100, the head-mounted display 200, the eye health platform management server 300, and the mobile type computing device 400.

In detail, the eye health measurement system according to an embodiment may be implemented based on an eye health measurement device, that is, the eye health measuring instrument 100 and the head-mounted display 200. (First system mode)

In detail, in an embodiment, the eye health measurement system may provide an eye health measurement service based on the eye health measuring instrument 100 implementing the eye health measurement service and the head-mounted display 200 implementing a virtual reality environment related to the eye health measurement service.

In another embodiment, the eye health measurement system may be implemented based on the head-mounted display 200 and the eye health platform management server 300.

### (Second system mode)

In detail, in another embodiment, the eye health measurement system may provide an environment in which the eye health measurement service may operate in the head-mounted display 200 based on the eye health platform management server 300, and provide the eye health measurement service based on the head-mounted display 200 provided with this operating environment.

In yet another embodiment, the eye health measurement system may be implemented based on the eye health measuring instrument 100 and a web server. (Third system mode)

In detail, in yet another embodiment, the eye health measurement system may provide an eye health measurement service online in conjunction with the eye health measuring instrument 100 with an external web server.

In another embodiment, the eye health measurement system may be implemented based on the eye health measurement device, in other words, that is, the eye health measuring instrument 100, the head-mounted display 200, and the mobile type computing device 400.

### (Fourth system mode)

In other words, in an embodiment, the eye health measurement system may provide an eye health measurement service based on the eye health measuring instrument 100 implementing the eye health measurement service, the head-mounted display 200 implementing a virtual reality environment related to the eye health measurement service, and the mobile type computing device 400 providing an eye health condition measurement result as a graphic image.

The virtual reality-based eye health measurement system according to an embodiment of the present invention may be implemented in any of the four system modes described above, but hereinafter, for effective description, description will be made based on the fourth system mode implemented based on the eye health measuring instrument 100, the head-mounted display 200 (in other words, an eye health measurement device), and the mobile type computing device 400.

Hereinafter, a method in which the processor 175 of the eye health measuring instrument 100 according to an embodiment of the present invention is linked with the head-mounted display 200 and the mobile type computing device 400 to provide an eye health measurement service based on virtual reality will be described in detail with reference to the accompanying drawings.

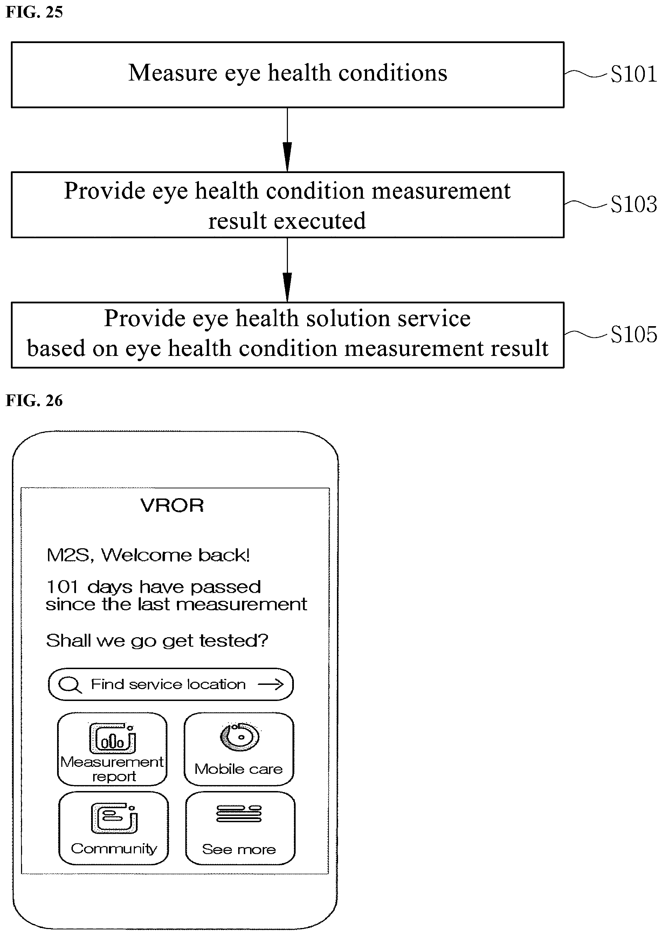

FIG. 25 is a flowchart illustrating a method for virtual reality-based eye health measurement according to an embodiment of the present invention.

Referring to FIG. 25, the processor 175 of the eye health measuring instrument 100 according to an embodiment of the present invention may measure eye health conditions based on virtual reality. (S201)

In detail, in an embodiment, the processor 175 may provide a plurality of eye health measurement methods based on the eye health measurement service.

In an embodiment, the processor 175 may provide an eye health measurement method including eyesight measurement, visual field measurement, astigmatism measurement, macular degeneration measurement, color blindness measurement, stereopsis measurement, diplopia measurement, contrast sensitivity measurement, extrinsic ocular muscle measurement, and/or dynamic eyesight measurement content based on a virtual reality image.

In addition, in an embodiment, the processor 175 may determine at least one eye health measurement method among a plurality of provided eye health measurement methods.

In an embodiment, the processor 175 may determine at least one eye health measurement method to be performed on a subject based on a result of a preliminary eye health condition survey of the subject.

In addition, in an embodiment, the processor 175 may determine at least one eye health measurement method to be performed on a corresponding subject based on an input of a user (in an embodiment, a subject and/or measurer).

As described above, the processor 175 that has determined at least one eye health measurement method to be performed on a subject may execute an eye health condition measurement process based on the determined at least one eye health measurement method.

In the following embodiment, for effective description, among a plurality of eye health measurement methods, stereopsis measurement content is set as the first eye health measurement method, and eye health measurement content is set as a second eye health measurement method to execute eye health measurement. However, this is merely an embodiment, and according to an embodiment, at least one eye health condition measurement based on various pieces of eye health measurement content (for example, visual field measurement, astigmatism measurement, macular degeneration measurement, color blindness measurement, diplopia measurement, contrast sensitivity measurement, extrinsic ocular muscle measurement and/or dynamic eyesight measurement) may be performed.

In an embodiment, the processor 175 may measure eye health conditions of a subject based on the determined first eye health measurement method.

In detail, in an embodiment, the processor 175 may set stereopsis measurement content as the first eye health measuring method, and may measure eye health conditions of a subject based thereon.

Herein, the stereopsis measurement content according to an embodiment may be content that measures whether an accurate depth may be recognized using the parallax of both eyes.

More specifically, in an embodiment, the processor 175 may execute eye health measurement based on stereopsis measurement content.

Further, the processor 175 may display at least three optotypes based on an virtual reality image in conjunction with the head-mounted display 200 based on the executed stereopsis measurement content.

For example, the processor 175 may display four optotypes arranged in a line on an virtual reality image.

In addition, in an embodiment, the processor 175 may provide a stereopsis measurement interface (a first eye health condition measurement interface) capable of selecting a displacement optotype, which is an optotype having a different depth from other optotypes, among a plurality of displayed optotypes.

In detail, the processor 175 may display a plurality of optotypes including one displacement optotype on a virtual reality image, and may provide a stereopsis measurement interface for selecting a displacement optotype among the plurality of displayed optotypes.

In this connection, in an embodiment, the processor 175 may adjust the position and/or size of a first optotype to be displayed as a displacement optotype in order to implement the displacement optotype having a different depth from other optotypes on a virtual reality image.

In an embodiment, a detailed description of measuring, by the processor 175, the eye health measurement based on the stereopsis measurement content will be replaced with a description of the stereopsis measurement content described in the method for measuring eye health based on virtual reality.

In an embodiment, the processor 175 may measure the eye health conditions of a subject based on the second eye health measurement method determined based on the survey interface.

In detail, in an embodiment, the processor 175 may set the eyesight content as the second eye health measurement method, and may measure the eye health conditions of a subject based thereon.

Herein, the eyesight content according to an embodiment may be content for measuring eyesight and focus ability.

In an embodiment, a detailed description of measuring, by the processor 175, the eye health measurement based on the eyesight measurement content will be replaced with a description of the eyesight measurement content described in the method for measuring eye health based on virtual reality.

In addition, referring further to FIGS. 19A and 19B, in an embodiment, the processor 175 may provide a result of eye health condition measurement (in an embodiment, stereopsis measurement and/or eyesight measurement) executed as described above. (S203)

In other words, in an embodiment, the processor 175 may provide eye health condition information, which is result information on at least one piece of eye health condition measurement executed.

Herein, the eye health condition information according to an embodiment is information providing an analysis result of eye health conditions of a subject estimated based on an eye health measurement service. In an embodiment, the eye health condition information may include result scores for each piece of eye health condition measurement performed on a subject (in an embodiment, stereopsis measurement result scores and/or eyesight measurement result scores), an integrated score calculated based on at least one result score (for example, the average score calculated based on the stereopsis measurement result score and the eyesight measurement result score), and/or predictive suspected disease information acquired based on an eye health measurement result (for example, macular degeneration, astigmatism, and glaucoma).

In detail, in an embodiment, the processor 175 may acquire eye health condition information for a subject based on an input of the subject to the eye health condition measurement interface (in an embodiment, a stereopsis measurement interface and/or an eyesight measurement interface) provided in an eye health condition measurement process.

In this connection, the processor 175 may provide more precisely analyzed result information based on a plurality of pieces of eye health condition information when there is a plurality of pieces of eye health condition measurement data depending on a subj ect.

For example, the processor 175 performs an analysis based on a plurality of pieces of eye health condition information acquired by measuring eye health condition multiple times, thereby more accurately predicting a disease such as glaucoma (in other words, a disease that needs to be identified over a long period of time), which is difficult to accurately understand only with a one-time measurement of eye health conditions.

Thus, the processor 175 may further improve the performance of the eye health measurement service.

In addition, in an embodiment, the processor 175 may output the acquired eye health condition information based on the display portion 150 as shown in FIG. 19A.

In addition, the processor 175 may output eye health condition information of a subject through the display unit 220 in conjunction with the head-mounted display 200 as shown in FIG. 19B.

In addition, in an embodiment, the processor 175 may store and manage the acquired eye health condition information by making the same into a database for each subject.

Thus, the processor 175 may digitize result data of eye health condition measurement and conveniently manage the same.

In addition, in an embodiment, the processor 175 may transmit and provide the acquired eye health condition measurement result data, in other words, eye health condition information, to the eye management application 411 of the mobile type computing device 400.

In detail, in an embodiment, the processor 175 may transmit the eye health condition information to the eye management application 411 of the mobile type computing device 400, and may implement and provide corresponding eye health condition information and various pieces of content information based on the eye health condition information in various ways based on the functional operation of the eye management application 411 receiving the same.

FIG. 26 is an example of a state in which an eye health solution service is provided in a mobile type computing device according to an embodiment of the present invention.

In more detail, referring to FIG. 26, in an embodiment, the processor 175 may be linked with the eye management application 411 of the mobile type computing device 400 that has received the eye health condition measurement result, and may provide an eye health solution service based on the corresponding eye health condition measurement result. (S205)

Herein, the eye health solution service according to an embodiment may be a service that provides various pieces of service content for customized eye health management optimized for a measurement subject based on the eye health condition measurement result acquired as described above from a diversified perspective, based on the eye management application 411 of the mobile type computing device.

In an embodiment, the eye health solution service may include a measurement report service, an eye care service, an eye health knowledge service, and/or an expert consultation service.

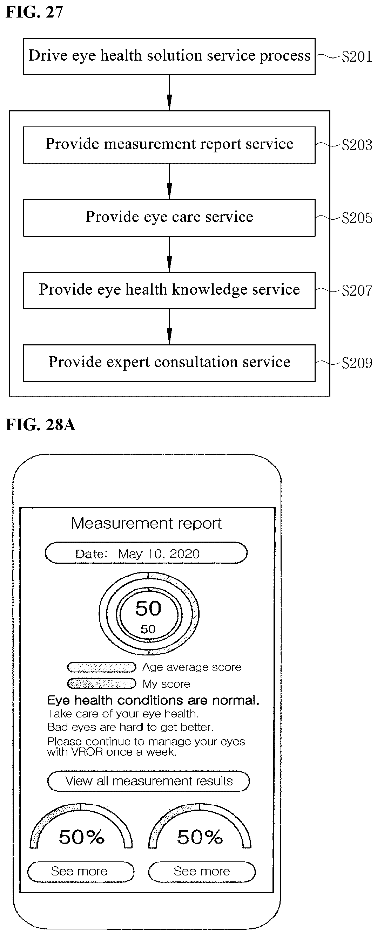

FIG. 27 is a conceptual diagram for explaining a method of providing an eye health solution service in a mobile type computing device according to an embodiment of the present invention.

Hereinafter, with reference to FIG. 27, a method for providing an eye health solution service by the eye management application 411 of the mobile type computing device 400 receiving eye health condition information from the processor 175 will be described in detail.

In detail, in an embodiment, the eye management application 411 of the mobile type computing device 400 that receives eye health condition information from the processor 175 of the eye health measuring instrument 100 may drive an eye health solution service process. (S301)

In an embodiment, the eye management application 411 may drive an eye health solution service process based on an input of a subject to the corresponding application.

In addition, in an embodiment, the eye management application 411 may provide a measurement report service based on the driven eye health solution service. (S303)

Herein, the measurement report service according to an embodiment may be a service that displays the eye health measurement result (in an embodiment, eye health condition information) executed for a subject in a graphic image so that the eye health condition index of the subject may be easily and intuitively understood.

In an embodiment, the eye management application 411 may provide the aforementioned measurement report service based on measurement report content and/or eye movement index content.

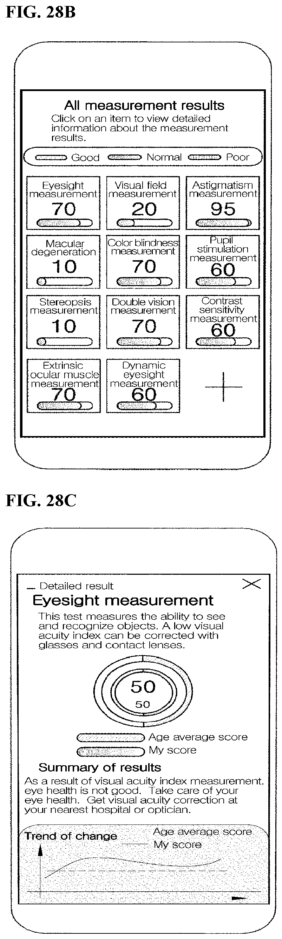

FIGS. 28A, 28B and 28C are examples of diagrams for explaining measurement report content according to an embodiment of the present invention.

In detail, referring to FIGS. 28A, 28B, and 28C, in an embodiment, the eye management application 411 may provide a measurement report service based on measurement report content.

Herein, the measurement report content according to an embodiment may be content that outputs eye health condition information and detailed result information based on the eye health condition information as graphic images and provides the same.

In this connection, the detailed result information according to an embodiment may include a measurer's comment on the eye health condition information and/or a change trend graph of the eye health condition information according to multiple rounds of eye health condition measurement.

In more detail, in an embodiment, the eye management application 411 may acquire eye health condition information and detailed result information for each of the eye health measurement methods executed at least once.

In addition, the eye management application 411 may generate measurement report content that provides graphic images of the acquired eye health condition information and detailed result information.

Specifically, the eye management application 411 may generate measurement report content by graphically imaging result scores for each piece of eye health condition measurement (for example, stereopsis measurement result scores and/or eyesight measurement result scores), an integrated score calculated based on at least one result score (for example, the average score calculated based on the stereopsis measurement result score and the eyesight measurement result score), and/or predictive suspected disease information acquired based on an eye health measurement result (for example, macular degeneration, astigmatism, and glaucoma).

In addition, the eye management application 411 may generate measurement report contents by graphically imaging comments for each eye health measurement method (for example, a result summary comment by a measurer) and/or a graph of an eye health condition change trend.

In addition, the eye management application 411 may provide a measurement report service by outputting the measurement report content generated as above.

As such, the eye management application 411 generates and provides the measurement report content based on the acquired eye health condition information, so that a subject may easily and intuitively recognize the measurement result of his or her own eye health condition.

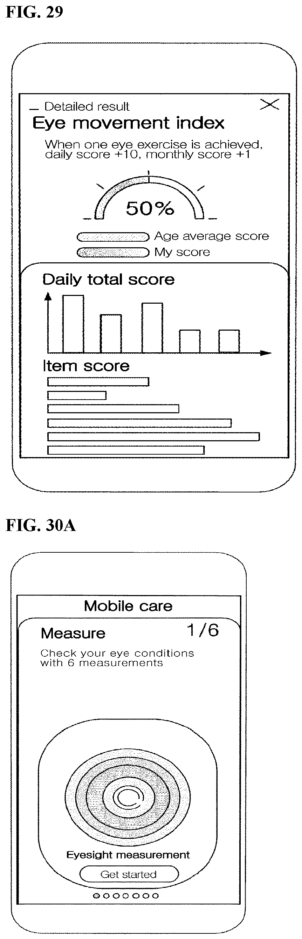

FIG. 29 is an example of a diagram for explaining eye movement index content according to an embodiment of the present invention.

In addition, referring to FIG. 29, in an embodiment, the eye management application 411 may provide a measurement report service based on the eye movement index content.

Herein, the eye movement index content according to an embodiment may be content that measures an eye movement amount of a subject based on an eye care service described later, and generates and provides eye movement information based thereon.

In an embodiment, the eye management application 411 may provide an eye movement index graph, daily eye movement amount score information, and/or eye movement item-specific score information for a subject as eye movement amount information based on the eye movement index content.

In detail, the eye management application 411 may calculate a progress rate for at least one piece of simple eye movement content executed for a subject among at least one or more pieces of simple eye movement content provided based on an eye care service described later.

For example, the eye management application 411 may calculate a progress rate for each piece of simple eye movement content based on a required time for each of at least one piece of simple eye movement content executed.

In addition, in an embodiment, the eye management application 411 may acquire an eye movement amount for the corresponding subject based on the calculated progress rate.

For example, the eye management application 411 may cause an eye movement amount for a subject to be determined in proportion to the calculated progress rate.

Further, the eye management application 411 may determine whether progress is completed (achieved) for each piece of simple eye movement content based on the progress rate calculated as above.

For example, the eye management application 411 may determine that the progress of first simple eye movement content is completed when the progress rate of the first simple eye movement content satisfies a predetermined criterion (for example, 100%).

In addition, in an embodiment, the eye management application 411 may generate and provide eye movement index content based on the calculated eye movement amount and/or progress completion (achievement).

For example, the eye management application 411 may generate an eye movement index graph according to whether the simple eye movement content has been completed.

For example, the eye management application 411 may calculate an eye movement index score by adding 10 points each time when one of a plurality of pieces of simple eye movement content is completed.

In addition, in an embodiment, the eye management application 411 may generate an eye movement index graph based on an eye movement index score calculated as above.

In an embodiment, in an embodiment, the eye management application 411 may generate and provide eye movement index content based on the generated eye movement index graph.

As such, the eye management application 411 may implement a benefit of motivation for encouraging eye movement by providing eye movement index content that provides a graphic image of the eye movement status of a subject.

In an embodiment, the eye management application 411 may provide an eye care service based on the driven eye health solution service. (S305)

Herein, the eye care service according to an embodiment may be a service that enables eye care to easily measure eye health conditions and perform eye movement anytime and anywhere using the mobile type computing device 400 in which the eye management application 411 is installed.

In detail, in an embodiment, the eye management application 411 may provide an eye care service based on simple eye measurement content and/or simple eye movement content.

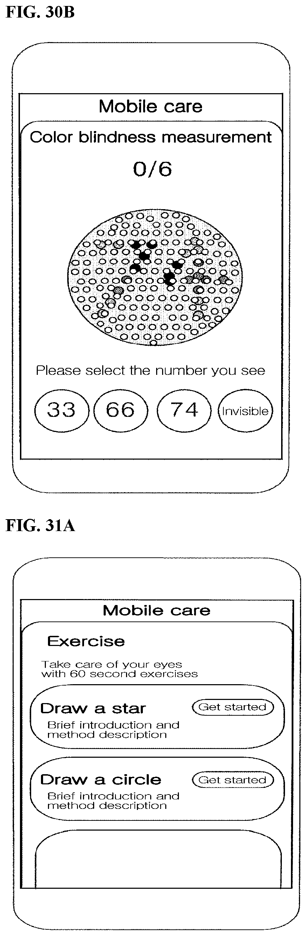

FIGS. 30A and 30B are examples of diagrams for explaining simple eye measurement content according to an embodiment of the present invention.

Referring to FIGS. 30A and 30B, in an embodiment, the eye management application 411 may provide an eye care service based on simple eye measurement content.

Herein, the simple eye measurement content according to an embodiment may be content that provides an environment in which eye health conditions may be easily measured anytime and anywhere, based on the eye management application 411 of the mobile type computing device 400.

In detail, in an embodiment, the eye management application 411 may provide a light version of an eye health measurement method process that may easily measure eye health conditions using the mobile type computing device 400 based on the simple eye measurement content.

For example, the eye management application 411 may provide a light version of an eyesight measurement process and/or a color blindness measurement process based on a 2-dimensional graphic image.

As such, the eye management application 411 provides simple eye measurement content that may easily and lightly measure eye health conditions without time or space limitations, so that a subject can conveniently perform self-diagnosis to understand eye health conditions using a more specialized and systematic method, and thus, the reliability of the self-diagnosis can be improved.

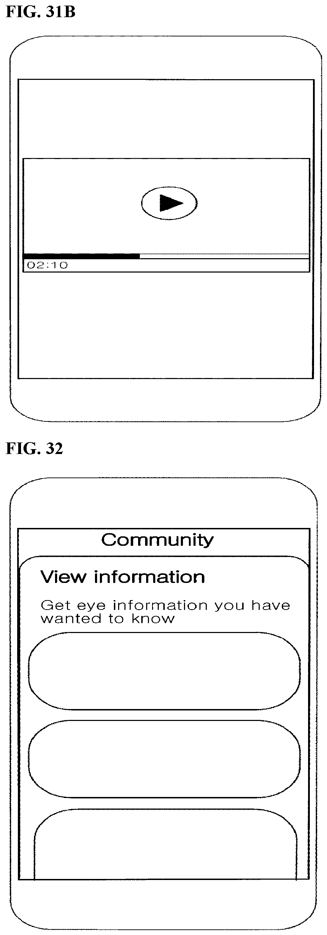

FIGS. 31A and 31B are examples of diagrams for explaining simple eye movement content according to an embodiment of the present invention.

In addition, referring to FIGS. 31A and 31B, the eye management application 411 may provide an eye care service based on simple eye movement content.

Herein, the simple eye movement content according to an embodiment may be content that provides an environment in which an eye movement may be easily performed anytime and anywhere, based on the eye management application 411 of the mobile type computing device 400.

In detail, in an embodiment, the eye management application 411 may provide at least one simple eye movement process based on the simple eye movement content.

Here, the simple eye movement process may be a process of providing graphic images for inducing eye activities of a subject for the purpose of improving eye health conditions.

For example, the simple eye movement process may include a star drawing movement process and/or a circle drawing movement process based on eyeball motion.

More specifically, in an embodiment, the eye management application 411 may execute one of at least one simple eye movement process based on an input of a subject.

In addition, the eye management application 411 may provide a simple eye movement process selected and executed based on an input of a subject based on a 2-dimensional graphic image.

In other words, in an embodiment, the eye management application 411 may implement a simple eye movement for inducing eye activities of a subject based on a 2-dimensional graphic image.

Thereafter, in an embodiment, the eye management application 411 may measure an eye movement amount of a subject according to the execution of the simple eye movement process based on the aforementioned eye movement index content, and generate and provide eye movement amount information based thereon.

As such, the eye management application 411 provides the simple eye movement content that may simply perform eye movements anytime and anywhere. Accordingly, it is possible to perform eye movement conveniently without time or space limitations without a separate device or effort to perform eye movement, thereby reducing the cost for eye care and inducing to participate more actively and positively in eye movement.

In an embodiment, the eye management application 411 may provide an eye health knowledge service based on the driven eye health solution service. (S307)

Herein, the eye health knowledge service according to an embodiment may be a service that provides a process for conveniently accessing various pieces of information related to eye health.

In an embodiment, the eye management application 411 may provide an eye health knowledge service based on eye health-related knowledge content and/or ophthalmic institution information content.

FIG. 32 is an example of a diagram for explaining knowledge content related to eye health according to an embodiment of the present invention.

Referring to FIG. 32, the eye management application 411 may provide an eye health knowledge service based on eye health-related knowledge content.

Herein, the eye health-related knowledge content according to an embodiment may be content that provides a community service that provides various pieces of knowledge related to eye health (for example, a method of escaping dry eye syndrome and/or a method of protecting eyes from blue light).

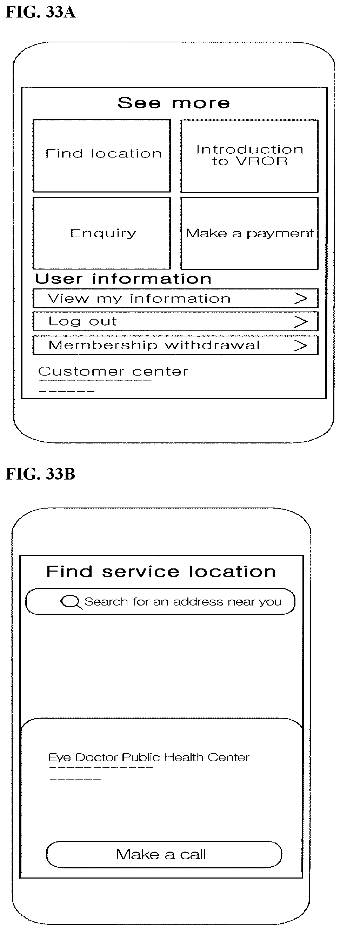

FIGS. 33A and 33B are examples of diagrams for explaining ophthalmic institution information content according to an embodiment of the present invention.

In addition, referring to FIGS. 33A and 33B, in an embodiment, the eye management application 411 may provide an eye health knowledge service based on ophthalmic institution information content.

Herein, the ophthalmic institution information content according to an embodiment may be content that provides various pieces of information (for example, ophthalmic institution location information, contact information, introduction information, nearest ophthalmic institution information and/or eye health measurement result customized ophthalmic hospital information) related to ophthalmic institutions (in an embodiment, an ophthalmic hospital and/or an institution providing an eye health measurement service based on an eye health measurement device).

In other words, in an embodiment, the eye management application 411 allows a subject to easily acquire and utilize various pieces of information related to eye health, based on the eye health knowledge service including the aforementioned content (in an embodiment, eye health-related knowledge content and/or ophthalmic institution information content).

Thus, the eye management application 411 can effectively reduce the cost required for information search for eye health care of a subject.

In an embodiment, the eye management application 411 may provide an expert consultation service based on the driven eye health solution service. (S309)

Herein, the expert consultation service according to an embodiment may be a service providing a communication function with an eye expert (for example, an ophthalmologist and/or professor).

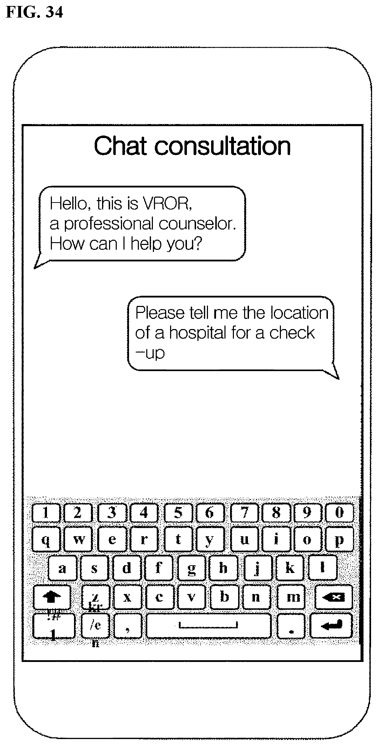

FIG. 34 is an example of a diagram for explaining expert consultation content according to an embodiment of the present invention.

Referring to FIG. 34, in an embodiment, the eye management application 411 may provide an expert consultation service based on expert consultation content.

Herein, the expert consultation content according to an embodiment may be content that provides an environment in which communication with an eye expert (for example, an ophthalmologist and/or professor) may be implemented.

In an embodiment, the expert consultation content may provide a real-time chatting consultation interface, a mail consultation interface, and/or a visit reservation consultation interface with an eye expert.

As such, the eye management application 411 enables communication with an eye expert using the eye management application 411 installed on the mobile type computing device 400 of a subject. Accordingly, it is possible to simplify the procedures required for communication with an eye specialist and simultaneously minimize the required time or cost, thereby enabling prompt and accurate eye health care.

As described above, the virtual reality-based eye health measurement system according to an embodiment of the present invention provides the eye health condition measurement result of a measurement subject acquired based on virtual reality based on an eye health measurement computing device of the measurement subject (for example, a mobile type computing device and/or a desktop type computing device). Accordingly, it is possible to easily track, observe, and care for eye health conditions in life, and thus, it is possible to reduce the cost or effort required for eye health management.

In addition, the virtual reality-based eye health measurement system according to an embodiment of the present invention provides an eye health solution service that assists customized eye health management optimized for a measurement subject from a diversified perspective based on the eye health condition measurement result. Accordingly, it is possible to conveniently manage and care for eye health in various aspects, and thus, it is possible to induce the measurement subject to participate more actively in eye health management also in daily life.

Embodiments of the present invention described above may be implemented in the type of program commands the may be executed through various computer components, and may be recorded on a computer-readable recording medium. The computer-readable recording medium may include program commands, data files, and data structures individually or in combinations thereof. The program commands that are recorded on a computer-readable recording medium may be those specifically designed and configured for the present invention or may be those available and known to those engaged in computer software in the art. The computer-readable recording medium includes magnetic media such as hard disks, floppy disks, and magnetic media such as a magnetic tape, optical media such as CD-ROMs and DVDs, magneto-optical media such as floptical disks, and hardware devices specifically configured to store and execute program commands, such as ROM, RAM, and flash memory. The program commands include not only machine language codes compiled by a compiler, but also high-level language code that may be executed by a computer using an interpreter. A hardware device may be changed into one or more software module to perform the processes according to the present invention, and vice versa.

### Industrial Applicability

An embodiment of the present invention relates to a system including a head-mounted display for displaying VR and a method for measuring eye health using the same, and thus is industrially applicable.

## Claims

1. A method for virtual reality-based eye health measurement performed by a processor of an eye health measuring instrument, which is linked with a head-mounted display, the method comprising:
providing an eye health condition survey interface;
determining an eye health measurement method based on the provided survey interface;
measuring eye health conditions with the determined eye health measurement method;
displaying the measurement result of the eye health conditions; and
providing prescription content based on the measurement result of the eye health conditions.

2. The method of claim 1, wherein the determination of the eye health measurement method comprises outputting at least one of a plurality of pieces of eye health measurement content based on an input of the measurement subject to the survey interface to determine an eye health measurement method for measuring eye health of the measurement subject, wherein the eye health measurement content is content that provides an eye health condition measurement process for the measurement subject according to a predetermined parameter.

3. The method of claim 2, wherein the determination of the eye health measurement method further comprises:
calculating an eye health score for each piece of the eye health measurement content based on the input of the measurement subject to the survey interface;
predicting a suspected disease based on the calculated eye health score; and
determining the eye health measurement method based on the predicted suspected disease.

4. The method of claim 1, wherein:
the determination of the eye health measurement method comprises determining stereopsis measurement content as the eye health measurement method; and
the measurement of the eye health conditions comprises:
displaying at least three optotypes on a virtual reality image based on the stereopsis measurement content;
determining any one of the at least three optotypes as a displacement optotype having a depth different from other optotypes and displaying each optotype;
providing a stereopsis measurement interface for selecting the displacement optotype; and
acquiring a stereopsis measurement result based on an input of the measurement subject to the provided stereopsis measurement interface.

5. The method of claim 4, wherein the displaying of the displacement optotype comprises:
determining one of the at least three optotypes as a first optotype that is a displacement optotype;
calculating an angle of stereopsis based on the depth to determine output positions of a 1-1 optotype representing the first optotype in a first display region corresponding to a left eye region of the measurement subject and a 1-2 optotype representing the first optotype in a second display region corresponding to a right eye region of the measurement subject according to the calculated angle of stereopsis; and
displaying the 1-1 optotype and the 1-2 optotype at the determined output positions on the first display region and the second display region, respectively, to display the displacement optotype.

6. The method of claim 1, wherein:
the determination of the eye health measurement method comprises determining eyesight measurement content as the eye health measurement method; and
the measurement of the eye health conditions comprises:
displaying a predetermined eyesight measurement table on a virtual reality image based on the eyesight measurement content;
providing an eyesight measurement interface for selecting a target symbol from among a plurality of symbols in the displayed eyesight measurement table; and
acquiring an eyesight measurement result based on an input of the measurement subject to the provided eyesight measurement interface.

7. The method of claim 1, wherein:
the provision of the prescription content comprises providing a first eye healing image for assisting a movement of intrinsic ocular muscles or extrinsic ocular muscles; and
the provision of the first eye healing image comprises:
displaying objects implemented with at least two different depths on a virtual reality image;
selecting any one of the displayed objects as a first object;
performing blur processing on remaining objects other than the selected first object;
changing and outputting a position and depth of the selected first object based on a preset criterion; and
converting the first object into any one of the remaining objects according to a predetermined criterion.

8. A method for virtual reality-based eye health measurement performed by a processor of an eye health measuring instrument, which is linked with a head-mounted display, the method comprising:
measuring eye health conditions of a measurement subject with a virtual reality-based eye health measurement method;
controlling to display eye health condition information of the measurement subject, which is a result of the measured eye health conditions;
determining an eye healing image, which is virtual reality content to be provided to the measurement subject, based on the generated eye health condition information; and
controlling to output the determined eye healing image to the measurement subject.

9. The method of claim 7, wherein the provision of the determined eye healing image to the measurement subject comprises controlling a plurality of virtual objects to be output with at least one of depth and visibility different.

10. The method of claim 9, wherein the provision of the determined first eye healing image to the measurement subject comprises:
displaying a first virtual object and a second virtual object;
guiding to gaze at the first virtual object; and
controlling a depth and position of the first virtual object to be changed and displayed according to a predetermined criterion.

11. The method of claim 9, further comprising controlling to sense a change in an eyeball of the measurement subject while changing and displaying the depth and position of the first virtual object according to a predetermined criterion.

12. The method of claim 9, wherein the displaying of the first virtual object and the second virtual object comprises controlling the first virtual object to be displayed with relatively higher visibility than the second virtual object.

13. The method of claim 12, wherein the displaying of the first virtual object and the second virtual object comprises:
changing visibility of a virtual object to display the second virtual object with relatively higher visibility than the first virtual object; and
guiding to gaze at the second virtual object.

14. A device for virtual reality-based eye health measurement, the device comprising:
an eye health measuring instrument providing a virtual reality-based eye health condition measurement service; and
a head-mounted display outputting the virtual reality image,
wherein the eye health measuring instrument comprises:
a body forming a body of the eye health measuring instrument;
a sensor portion sensing an inflow or outflow of the head-mounted display to a head-mounted display accommodation portion disposed on the body;
a disinfection portion disinfecting the head-mounted display introduced into the accommodation portion; and
a processor controlling the sensor portion to acquire protection portion sensing information including at least one of position, area, direction, and angle information of a facial contact protection portion of the head-mounted display introduced into the accommodation portion, and tilting the disinfection portion toward the facial contact protection portion based on the acquired protection portion sensing information.

15. The device of claim 14, wherein the eye health measuring instrument further comprises a charging portion for charging the head-mounted display introduced into the accommodation portion.

16. The device of claim 14, wherein:
the eye health measuring instrument further comprises a driving portion for assisting a tilting operation of the disinfection portion;
the driving portion comprises a first driving portion disposed on one side surface of the accommodation portion and a second driving portion disposed on the other side surface of the accommodation portion; and
the processor controls a vertical movement of at least one of the first driving portion and the second deriving portion to tilt at least a portion of a first disinfection portion disposed on one side surface of the accommodation portion, a second disinfection portion disposed on the other side surface of the accommodation portion, and a third disinfection portion disposed on an upper surface of the accommodation portion.

17. The device of claim 14, wherein:
the sensor portion comprises a user sensing unit that determines whether a user approaches the eye health measuring instrument by a predetermined distance or less; and
the processor automatically executes the eye health condition measurement service based on user proximity information acquired from the user sensing unit.

18. The device of claim 14, wherein:
the disinfection portion comprises at least one lamp of an ultraviolet lamp (UV lamp) and an LED lamp (LED LAMP); and
the processor controls the lamp of the disinfection portion tilted toward the facial contact protection portion to irradiate light toward the facial contact protection portion.

19. The device of claim 14, wherein the processor generates sterilization and disinfection information, which is information obtained by calculating a degree of sterilization of the facial contact protection portion by the disinfection portion according to a predetermined criterion.

20. The device of claim 19, wherein:
the eye health measuring instrument further comprises a display portion that outputs a graphic image of the eye health condition measurement service; and
the processor controls the display portion to output the sterilization and disinfection information.
